# EUROPEAN PATENT SPECIFICATION

(11) **EP 4 442 186 B1**
(45) Date of publication and mention of the grant of the patent: **04.02.2026**
(21) Application number: 22907789.6
(22) Date of filing: 02.12.2022
(51) Int. Cl.: A47L 23/20, A47B 61/04, F26B 7/00, F26B 21/00, F26B 3/02, F26B 9/00, A61L 2/07

(54) **SHOE CARE DEVICE AND SHOE CARE SET INCLUDING SAME**
SCHUHPFLEGEVORRICHTUNG UND SCHUHPFLEGESET DAMIT
DISPOSITIF D'ENTRETIEN DE CHAUSSURES ET ENSEMBLE D'ENTRETIEN DE CHAUSSURES LE COMPRENANT

(30) Priority: 17.12.2021 KR 20210181898
(43) Date of publication of application: 09.10.2024
(73) Proprietor: LG Electronics Inc., Yeongdeungpo-gu Seoul 07336 (KR)
(72) Inventor: KIM, Jae Young, Seoul 08592 (KR); NAM, Bo Hyun, Seoul 08592 (KR); SONG, Seung Hyun, Seoul 08592 (KR)
(74) Representative: Schornack, Oliver
(86) International application number: PCT/KR2022/019472
(87) International publication number: WO 2023/113320

(56) References cited:
- WO-A1-2017/003170
- CN-U- 211 862 183
- CN-U- 214 963 588
- CN-U- 214 963 588
- KR-A- 20000 009 653
- KR-A- 20200 001 053
- KR-A- 20200 001 053
- KR-A- 20210 030 856
- KR-A- 20210 113 002
- KR-B1- 101 037 245
- KR-U- 20200 002 534

## Description

The present invention relates to a shoe care device and shoe care set including same, and more particular, to a shoes care device and shoe care set including same which treats shoes by air circulation.

### [Background Art]

Shoes may get wet by a wearer's sweat, external contaminants, or rain or snow. Wearing such shoes may make the wearer uncomfortable, and in the condition, germs may also breed or stink in the shoes.

Accordingly, there is an increasing interest in a shoes care device 1, which removes germs and odors by performing a predetermined treatment on the shoes so that a user can comfortably wear the shoes all the times.

For the shoes care device, Korean Patent No. 1037245 (hereinafter, referred to as "Prior Document 1") discloses "Apparatus for sterilization disposal of shoes", which includes a main body, an ultraviolet emission module, and a deodorization module.

According to Prior Document 1 above, the shoes are disposed in a sterilization chamber of the main body, and the ultraviolet emission module is driven to remove germs and odors of the shoes. Furthermore, the air in the sterilization chamber is inhaled into a ventilation pipe and discharged to the outside of the main body through an exhaust port through the deodorization module.

Here, the deodorization module includes a deodorization column made up of materials such as zeolite, activated carbon, and charcoal, and removes contaminants from the air discharged from the inside of the main body to the outside by the deodorization column.

According to Prior Document 1 above, the air from which moisture has been removed by a deodorization module including zeolite and activated carbon can be discharged to the outside of the apparatus for sterilization disposal of shoes.

However, in Prior Document 1 above, since the air is discharged to the outside of the apparatus for sterilization disposal of shoes, the air that has not sufficiently removed moisture or odors may be discharged to the outside of the apparatus for sterilization disposal of shoes, and such air may be discharged to the inside where the wearer resides.

In addition, Korean Patent Unexamined Publication No. 10-2000-0009653 (hereinafter referred to as "Prior Document 2") discloses "The shoes cabinet for sanitation", which includes a main body, a far infrared radiation part, a circulation fan, an air circulation passage, and a sanitary filter portion.

According to Prior Document 2 above, while storing shoes in shoe closet, hygiene treatments such as dehumidification, sterilization, and deodorization can be performed on the shoes by far-infrared rays and a filter during the storing of the shoes.

Here, since the sanitary filter portion is filled with excellent adsorptive materials such as charcoal, it can serve to adsorb moisture in the process of ventilating air and filter bacteria, as well as to capture malodorous substances.

According to Prior Document 2 above, the air is circulated by a circulating fan in a shoe closet, and the sanitary filter portion is disposed on a circulation path of the air to remove germs and odors in the air.

However, since Prior Document 2 above does not consider a technology to prevent the reduction of the performance of the sanitary filter portion configured to remove moisture or odors, a user may not be satisfied because the shoes are not properly treated at the time of using a shoes care device.

As described above, for the shoes care device that removes germs or odors by treating the shoes in a predetermined manner, a task that has to be solved to ensure proper performance for shoes treatment while preventing the air used for dehumidification and deodorization from being exposed to the user is present in in the process of treating the shoes.

However, there is a limitation in that the conventional shoes care device cannot properly solve such a task.

In addition, when designing and manufacturing devices including a dehumidifier such as zeolite, it is necessary to consider how the dehumidification efficiency of a dehumidifying material can be further improved, whether the dehumidifying material can be effectively regenerated, whether water vapor generated during the use of the shoes care device and the regeneration of the dehumidifying material can be effectively removed or managed, whether moisture is left in an unintended area during use of the shoes care device and the regeneration of the dehumidifying, whether components are properly disposed in a limited space, and where the devices provides excellent use convenience. There is a need to develop a shoes care device that takes these matters into consideration.

In addition, the reduction of manufacturing costs, the convenience of manufacturing and assembling each component, and the convenience of maintenance needs to be considered in the development of the shoes care device.

In general, the upper surfaces of home appliances may be used to place various articles temporarily or for a certain period of time. The above-mentioned various articles may be home appliances associated with the home appliances defining the upper surfaces. However, since the upper surface of a conventional shoe care device has a simple flat shape, there is a risk that articles placed on the upper surface may be easily reversed or slip and fall to the floor during the process of opening the door, or due to external force or tilting of the shoe care device. In addition, when an article placed on the upper surface of the shoe care device is located above the door of the shoe care device door, there is a risk that the article may interfere with the opening and closing of the door of the shoe care device.

Shoes treated through the shoe care device may be stored or displayed on the upper surface of the shoe care device. At this time, the shoes treated through the shoe care device may be displayed in the state of being stored in a case with the inside visible.

Korean Patent Laid-open Publication No. 20-2020-0002534 (hereinafter, referred to as "Prior Document 3") discloses "Case for Displaying Shoes", and the shoe display case according to this includes a main body, a light emitting unit, a negative ion generator, a blower, and the like.

According to Prior Document 3, the front and side surfaces are open so that it is easy to identify shoes stored in the shoe display case, and an interior effect may be improved by creating lights in various colors through the light emitting unit.

Since Prior Document 3 described above does not consider stacking on the upper surface of the shoe care device or interfering with the opening and closing of the door or the shoe care device, there is a risk that various inconvenient situations may occur while a user uses a shoe display case stacked on the shoe care device. However, conventional shoe care devices and shoe display cases have limitations in that they cannot appropriately solve these problems. Therefore, there is a need for development of a shoe management device and a shoe display case in which the above problems are taken into consideration.

Document KR 2021 0030856 A (hereinafter, referred to as "Prior Document 4") describes a shoe care device and a shoe care set according to the preambles of independent claims 1 and 7.

### [Technical Problem]

In view of the foregoing, the present invention is to provide a shoe care device in which articles can be stacked on the upper surface thereof and interior aesthetics can be provided by the upper surfaces of a cabinet and a door that constitute the upper surfaces of the shoe care device.

In addition, the present invention is to provide a shoe care device in which, even when articles are stacked on the upper surface of the shoe care device, the door can be easily opened, and the articles stacked on the upper surface of the shoe care device can be prevented from being reversed or falling.

Furthermore, the present invention is to provide a shoe care set in which a visible shoe care device can be stably stacked on the upper surface of a shoe care device, and even if the visible shoe care device is stacked on the upper surface of the shoe care device, the opening and closing structures of the shoe care device and the visible shoe care device do not interfere with each other .

### [Technical Solution]

A shoe care device described herein may include a cabinet and a door.

The cabinet may include an inner cabinet and an outer cabinet.

The inner cabinet may provide an accommodation space therein. The inner cabinet may have a main opening that opens the accommodation space in a horizontal first direction.

The external cabinet may include an outer upper plate. The outer upper plate may define the upper surface of the cabinet.

The door may be configured to open and close the main opening. The door may be hinged to the cabinet.

The door may include an upper surface plate. A second concave groove may be provided in the upper surface plate. A user may apply force to the inner surface of the second concave groove to pull and open the door.

The shoe care device may include a nozzle. The nozzle may be provided in the accommodation space. The nozzle may spray forcibly blown air while inserted into the shoes.

A downwardly concave accommodation portion may be provided on the upper surface of the shoe care device. The accommodation portion have a symmetrical shape with reference to a reference plane parallel to the horizontal first direction and the vertical direction.

The cabinet and the door may together define the accommodation portion. The outer upper plate may define the upper surface of the cabinet. The upper surface plate may define the upper surface of the door. Therefore, the outer upper plate and the upper surface plate may together define an accommodation portion.

The accommodation portion may include an accommodation bottom portion, an accommodation extension portion, and an accommodation side portion.

The accommodation bottom portion may be provided on the upper surface of the cabinet. An article including a shoe care device may be seated on the accommodation bottom portion. The accommodation bottom portion may provide a surface on which an article can be seated.

The accommodation extension portion may be provided on the upper surface of the door. In the accommodation extension portion, a surface adjacent to the accommodation bottom portion may have the same height as the accommodation bottom portion.

The accommodation side portion may define an edge of the accommodation portion. The accommodation side portion may have a height higher than the accommodation bottom portion. The accommodation side portion may include a first accommodation side portion and a second accommodation side portion.

The first accommodation side portion may be provided on the upper surface of the cabinet along the edge of the accommodation portion. The first accommodation side portion may provide a surface connected to the accommodation bottom portion. The first accommodation side portion may include an eleventh accommodation side portion, a twelfth accommodation side portion, and a thirteenth accommodation side portion.

The eleventh accommodation side portion may be provided behind the accommodation bottom portion in the first direction. The eleventh accommodation side portion may be provided along the second direction. The second direction may be a horizontal direction perpendicular to the first direction.

The eleventh accommodation side portion may be raised upward in the third direction as it moves away from the accommodation bottom portion rearward in the first direction. The third direction may be a vertical direction perpendicular to the first direction.

The twelfth accommodation side portion may be provided on one side of the second direction with reference to the accommodation bottom portion. The twelfth accommodation side portion may be provided along the first direction. The twelfth accommodation side portion may be raised upward in the third direction as it moves away from the accommodation bottom portion toward one side in the second direction.

The thirteenth accommodation side portion may be provided on the other side in the second direction with reference to the accommodation bottom portion. The thirteenth accommodation side portion may be provided along the first direction. The thirteenth accommodation side portion may be raised upward in the third direction as it moves away from the accommodation bottom portion toward the other side in the second direction.

The second accommodation side portion may be provided on the upper surface of the door along the edge of the accommodation portion. The second accommodation side portion may provide a surface connected to the accommodation extension portion. The second accommodation side portion may include a twenty-first accommodation side portion, a twenty-second accommodation side portion, and a twenty-third accommodation side portion.

The twenty-first accommodation side portion may be provided in front of the accommodation extension portion in the first direction. The twenty-first accommodation side portion may be provided along the second direction. The twenty-first accommodation side portion may be raised upward in the third direction as it moves away from the accommodation extension portion forward in the first direction.

The twenty-second accommodation side portion may be provided on one side of the second direction based on the accommodation extension portion. The twenty-second accommodation side portion may be provided along the first direction. The twenty-second accommodation side portion may be raised upward in the third direction as it moves away from the accommodation extension portion toward one side in the second direction. The twenty-second accommodation side portion may provide a surface continuous with the surface of the accommodation extension portion.

The twenty-third accommodation side portion may be provided on the other side of the second direction with reference to the accommodation extension portion. The twenty-third accommodation side portion may be provided along the first direction. The twenty-third accommodation side portion may be raised upward in the third direction as it moves away from the accommodation extension portion toward the other side in the second direction. The twenty-second accommodation side portion may provide a surface continuous with the surface of the accommodation extension portion.

The second accommodation side portion and the accommodation extension portion may define a second concave groove. A user may apply force to the inner surface of the second concave groove to pull and open the door. The twenty-first accommodation side portion may be provided in front of the accommodation extension portion in the first direction.

The accommodation extension portion may be lowered toward the first direction. The twenty-first accommodation side portion may provide a surface continuous with the surface of the accommodation extension portion. Accordingly, the area of the second accommodation side portion to which an action force for opening the door is applied may increase downward in the third direction.

With reference to a direction parallel to the first direction, the gradient of the second accommodation side portion may be greater than the gradient of the first accommodation side portion.

The visible shoe care device can provide therein a display space to display shoes. The visible shoe care device may include a main body, a moving body, and a transparent window.

The main body may provide a display space.

The moving body may define a shape surrounding the lower portion of the main body. The moving body may be coupled to the main body to be slidable in a direction parallel to the first direction. The moving body may include a transparent window. The viewing window provide a surface in contact with the shoe care device.

The moving body may move rearward in the first direction to close the display space. The moving body may slide in the first direction to open the shoe care device.

A downwardly convex insertion portion may be provided on the bottom surface of the main body to be inserted into the accommodation portion. By inserting the insertion portion into the accommodation portion, the visible shoe care device can be stacked on the upper surface of the shoe care device. The insertion portion may include a seating portion, an insertion bottom portion, and an insertion side portion.

The seating portion may be seated on the accommodation bottom portion. A plurality of seating portions may be provided. The seating portions may protrude downward from the insertion bottom portion.

The insertion bottom portion may be spaced upward from the accommodation bottom portion by the seating portion. The insertion bottom portion may provide a horizontal surface parallel to a horizontal plane.

The insert side portion may be provided along the edge of the insertion bottom portion. The insert side portion may be connected to the insertion bottom portion. The insertion side portion may include a first insertion side portion, a second insertion side portion, a third insertion side portion, and a fourth insertion side portion.

The first insertion side portion may be provided behind the insertion bottom portion in the first direction. The first insertion side portion may be provided along the second direction. The first insertion side portion may be raised upward in the third direction as the first insertion side portion moves away from the insertion bottom portion rearward in the first direction.

At least a portion of the first insertion side portion may be lower than the upper end of the eleventh accommodation side portion. Therefore, when a user places the visible shoe care device or articles on the accommodation bottom portion, the first insertion side portion may provide an inclined surface corresponding to the eleventh accommodation side portion.

The second insertion side portion may be provided in front of the insertion bottom portion in the first direction. The second insertion side portion may be provided along the second direction. The second insertion side portion may be raised upward in the third direction as it moves away from the insertion bottom portion forward in the first direction.

The third insertion side portion may be provided on one side of the second direction with respect to the insertion bottom portion. The third insertion side portion may be provided along the first direction. The third insertion side portion may provide a vertical surface extending upward from the insertion bottom portion. At least a portion of the third insertion side portion may be higher than the upper end of the thirteenth accommodation side portion.

The fourth insertion side portion may be provided on the other side of the insertion bottom portion in the second direction. The fourth insertion side portion may be provided along the first direction. The fourth insertion side portion may provide a vertical surface extending upward from the insertion bottom portion. At least a portion of the fourth insertion side portion may be higher than the upper end of the twelfth accommodation side portion.

When the visible shoe care device or an article seated on the accommodation bottom portion is pushed toward the twelfth accommodation side portion or the thirteenth accommodation side portion by an external force or the tilting of the shoe care device, the third insertion side portion and the fourth insertion side portion may provide a surface that is in close contact with the twelfth accommodation side portion or the thirteenth accommodation side portion.

With reference to the first direction in the state in which the display space is closed, at least a portion of the second accommodation side portion may be located in front of the visible shoe care device. With reference to the first direction in the state in which the display space is closed, the second accommodation side portion and the moving body may be spaced apart from each other in the first direction. The user may open the door by inserting his or her finger into the second concave groove through a gap between the second accommodation side portion and the moving body.

A separation extension portion may be provided on the bottom surface of the main body. The separation extension portion may extend downward from the bottom surface of the main body. The separation extension portion may cause the insertion portion to be spaced apart downward from the bottom surface of the main body. The insertion portion may be spaced apart downward from the bottom surface of the main body by the separation extension portion. The separation extension portion and the insertion side portion may be connected to each other.

The moving body and the main body may be spaced apart from the second accommodation side portion in the third direction above the door. The user may open the door by easily inserting his or her finger into the second concave groove through a gap between the second accommodation side portion and the moving body.

### [Advantageous Effects]

In the shoe care device according to an embodiment of the present disclosure, a downwardly concave accommodation portion may be provided on the upper surface of the shoe care device. The accommodation portion may include an accommodation bottom portion, an accommodation extension portion, and an accommodation side portion. By configuring the accommodation side portion to have a height higher than the accommodation bottom portion at the edge of the accommodation portion, various articles seated on the accommodation bottom portion can stably maintain the state of being seated on the accommodation bottom portion.

In the shoe care device according to an embodiment of the present disclosure, the surface of the accommodation extension portion adjacent to the accommodation bottom portion may have the same height as the accommodation bottom portion. Therefore, even when a visible shoe care device or article seated on the accommodation bottom portion is slightly pushed toward the accommodation extension portion due to an external force or the tilting of the shoe care device, the visible shoe care device or article can be prevented from being reversed due to a difference in height between the accommodation bottom portion and the accommodation extension portion.

In the shoe care device according to an embodiment of the present disclosure, the first accommodation side portion may be raised upward toward the edge of the accommodation portion. Therefore, in the process of placing a visible shoe care device or article on the accommodation bottom portion by a user, the eleventh accommodation side portion may provide an inclined surface that guides the bottom surface of the visible shoe care device or article toward the accommodation bottom portion. Therefore, it is possible for the user to stably place the visible shoe care device or article on the accommodation bottom portion.

In the shoe care device according to an embodiment of the present disclosure, the first accommodation side portion may define a boundary where a visible shoe care device or an article is pushed to the outside of the accommodation bottom portion due to a difference in height from the accommodation bottom portion. Accordingly, various visible shoe care devices or articles seated on the accommodation bottom portion can stably maintain the state of being seated on the accommodation bottom portion.

In the shoe care device according to an embodiment of the present disclosure, the first accommodation side portion may provide a surface continuous with the surface of the accommodation bottom portion, and the gradient of the first accommodation side portion may increase toward the edge of the accommodation portion. Therefore, even when the visible shoe care device or article seated on the accommodation bottom portion is somewhat pushed toward the first accommodation side portion due to an external force or the tilting of the shoe care device, it is possible to suppress the visible shoe care device or article from being reversed. In addition, the first accommodation side portion can stably define the boundary where the visible shoe care device or article is pushed toward the opposite side of the door.

In the shoe care device according to an embodiment of the present disclosure, the surface of the accommodation extension portion adjacent to the accommodation bottom portion may have the same height as the accommodation bottom portion. Therefore, with reference to the state in which the door is closed, in the process of placing a visible shoe care device or article on the accommodation bottom portion by a user, the twenty-first accommodation side portion may provide an inclined surface that guides the bottom surface of the visible shoe care device or article toward the accommodation bottom portion via the accommodation extension portion.

In the shoe care device according to an embodiment of the present disclosure, when a visible shoe care device or article seated on the accommodation bottom portion is pushed toward the twenty-first accommodation side portion by an external force or the tilting of the shoe care device, the twenty-first accommodation side portion may define the boundary to which the visible shoe care device or article is pushed due to a difference in height from the accommodation bottom portion. Therefore, it is possible to prevent various visible shoe care devices or articles seated on the accommodation bottom portion from falling to the outside of the accommodation portion.

In the shoe care device according to an embodiment of the present disclosure, the twenty-first accommodation side portion may provide a surface continuous with the surface of the accommodation extension portion, and the gradient may increase toward the edge of the accommodation portion. Therefore, even when the visible shoe care device or article seated on the accommodation bottom portion is somewhat pushed toward the twenty-first accommodation side portion due to an external force or the tilting of the shoe care device, it is possible to suppress the visible shoe care device or article from being reversed.

In the shoe care device according to an embodiment of the present disclosure, the area of the second accommodation side portion to which an action force for opening the door is applied increases downward, so that the user's finger may come into contact with the second accommodation portion over a wide contact surface. Accordingly, since the acting force for opening the door is distributed across the contact surface, and the pressure acting on the unit area of the surface of the finger is reduced, the user may feel that the door is opened easily.

In the shoe care device according to an embodiment of the present disclosure, with reference to a horizontal direction, the gradient of the second accommodation side portion may be greater than the gradient of the first accommodation side portion. Accordingly, since the acting force applied to the twenty-first accommodation side portion via the user's finger increases in the horizontal component which is perpendicular to the rotation axis of the door, the user may feel that the door is opened easily.

In the shoe care device according to an embodiment of the present disclosure, since the first accommodation side portion and the second accommodation side portion have the same height along the edge of the accommodation portion, the accommodation portion may be observed as an integrated concave portion having the accommodation side portion of the constant height along the edge with reference to the state in which the door is closed, and the aesthetics of the upper surface of the shoe care device can be improved.

In the shoe care device according to an embodiment of the present disclosure, since the downwardly convex insertion portion is provided in the bottom surface of the main body so that the insertion portion can be inserted into the accommodation portion, a visible shoe care device can be stably stacked on the upper surface of the shoe care device.

In the shoe care device according to an embodiment of the present disclosure, when the visible shoe care device seated on the accommodation bottom portion is pushed toward the first accommodation side portion by an external force or the tilting of the shoe care device, a frictional force can be formed between the first insertion side portion and the first accommodation side portion. Therefore, various visible shoe care devices seated on the accommodation bottom portion can stably maintain the state of being seated on the accommodation bottom portion.

In the shoe care device according to an embodiment of the present disclosure, when the shoe care device is closed, at least a portion of the second accommodation side portion may be located in front of the moving body. Therefore, the user may easily open the door by inserting his or her finger into the second concave groove through a gap between the second accommodation side portion and the moving body.

In the shoe care device according to an embodiment of the present disclosure, the moving body and the main body may be vertically spaced apart from the second accommodation side portion above the door. Therefore, since the gap between the second accommodation side portion and the moving body is expanded in a direction approximately perpendicular to the direction in which the user's finger enters, it is possible for the user to open the door smoothly by easily inserting the finger into the second concave groove through the gap between the second accommodation side portion and the moving body.

In the shoe care device according to an embodiment of the present disclosure, since the moving body and the main body are vertically spaced apart from the second accommodation side portion above the door, the moving body can open and close the shoe care device in the front and rear direction above the insertion portion and the accommodation portion. Therefore, even when a visible shoe care device is stacked on the upper surface of the shoe care device, the visible shoe care device may not interfere with the opening and closing of the door.

### [Description of Drawings]

FIG. 1a is a perspective view illustrating a shoes care device according to one embodiment of the present invention.
FIG. 1b is a perspective view of the shoes care device of FIG. 1a, when viewed from another direction, illustrating a state in which a door is opened.
FIG. 2a is a perspective view illustrating a state in which a part of the door and an outer cabinet are removed from the shoes care device of FIG. 1b.
FIG. 2b is a perspective view illustrating a state in which the shoes care device illustrated in FIG. 2a is viewed from another direction.
FIG. 3 is a front view illustrating a state in which a door is removed from the shoes care device illustrated in FIG. 1b. FIG. 3 illustrates shoes accommodated in an inner cabinet together.
FIG. 4a is a cross-sectional view taken along line A-A' of the shoes care device illustrated in FIG. 3, FIG. 4b is a cross-sectional view taken along line B-B' of the shoes care device illustrated in FIG. 3, and FIG. 4c is a cross-sectional view taken along line C-C' of the shoes care device illustrated in FIG. 3. FIGS. 4a to 4c illustrate a form where a door is included in the shoes care device, and do not illustrate shoes.
FIG. 5 is a perspective view illustrating a state in which the door, the outer cabinet, and the inner cabinet are removed from the shoes care device according to one embodiment of the present invention.
FIG. 6 is a perspective view illustrating a part of a machine room of the shoes care device illustrated in FIG. 5.
FIG. 7a is a view illustrating a steam valve according to one embodiment of the present invention, and illustrating a connection relationship considering the movement of steam.
FIG. 7b is an exploded perspective view illustrating the steam valve illustrated in FIG. 7a.
FIG. 8a is a cross-sectional view taken along line D-D' of the shoes care device illustrated in FIG. 3. FIG. 8a illustrates a state in which the door is included in the shoes care device, and does not illustrate the shoes.
FIG. 8b is a view illustrating a state in which a main shelf is removed from the shoes care device illustrated in FIG. 8a.
FIG. 9 is a cross-sectional view taken along line E-E' of the shoes care device illustrated in FIG. 3. FIG. 9 illustrates a state in which the door is included in the shoes care device.
FIG. 10a is an exploded perspective view illustrating a drying module in the shoes care device according to one embodiment of the present invention. FIG. 10b is an exploded perspective view illustrating a partial configuration of the drying module at a part to which a damper is coupled.
FIG. 11 is a diagram illustrating a connection relationship between components and a flow of fluid in the shoes care device according to one embodiment of the present invention.
FIG. 12 is a perspective view illustrating the shoes care device and a visible shoes care device of a shoes management set according to one embodiment of the present invention.
FIG. 13 is a perspective view illustrating a state in which the visible shoes care device of the shoes management set of FIG. 12 is stacked on an upper surface of the shoes care device.
FIG. 14 is a perspective view of the shoes care device of FIG. 12 when viewed from another direction, illustrating a state in which the door is opened.
FIG. 15a is a perspective view illustrating the visible shoes care device of FIG. 12.
FIG. 15b is a view illustrating a state in which a display space of the visible shoes care device of FIG. 15 is opened
FIG. 16 is a perspective view illustrating a bottom surface of the visible shoes care device and the upper surface of the shoes care device, which constitutes the shoes management set of FIG. 12.
FIG. 17 is a partial side view of the shoes management set of FIG. 13.
FIG. 18a is a partial cross-sectional view of the shoes management set of FIG. 17 cut in an XZ plane and is a view illustrating Part A of FIG. 17.
FIG. 18b is a partial cross-sectional view of the shoes management set of FIG. 17 cut into the XZ plane and is a view illustrating Part B of FIG. 17.
FIG. 19 is a top plan view illustrating the upper surface of the shoes care device of FIG. 12.
FIG. 20 is a bottom view illustrating a bottom surfaces of the visible shoes care device of FIG. 12.
FIG. 21 is a partial front view of the shoes management set of FIG. 13.
FIG. 22a is a partial cross-sectional view of the shoes management set of FIG. 21 cut into an YZ plane and is a view illustrating Part C of FIG. 21
FIG. 22b is a partial cross-sectional view of the shoes management set of FIG. 21 cut into the YZ plane and is a view illustrating Part D of FIG. 21.

### [Modes for the Invention]

Hereinafter, exemplary embodiments disclosed herein will be described in detail with reference to the accompanying drawings, and like reference numerals designate like elements, and redundant description thereof will be omitted. Suffixes "module" and "unit or portion" for elements used in the following description are merely provided for facilitation of preparing this specification, and thus they are not granted a specific meaning or function. Wherever possible, the same reference numbers will be used throughout the drawings to refer to the same or like parts. In the following description, known functions or structures, which may confuse the substance of the present disclosure, are not explained. The accompanying drawings are used to help easily explain various technical features and it should be understood that the exemplary embodiments presented herein are not limited by the accompanying drawings. As such, the present disclosure should be construed to extend to any alterations, equivalents and substitutes in addition to those which are particularly set out in the accompanying drawings.

Although the terms first, second, and the like, may be used herein to describe various elements, these elements should not be limited by these terms. These terms are generally only used to distinguish one element from another.

When an element or layer is referred to as being "on," "engaged to," "connected to," or "coupled to" another element or layer, it may be directly on, engaged, connected, or coupled to the other element or layer, or intervening elements or layers may be present. In contrast, when an element is referred to as being "directly on," "directly engaged to," "directly connected to," or "directly coupled to" another element or layer, there may be no intervening elements or layers present.

The singular expressions include plural expressions unless the context clearly dictates otherwise.

It should be understood that the terms "comprises," "comprising," "includes," "including," "containing," "has," "having" or any other variation thereof specify the presence of stated features, integers, steps, operations, elements, and/or components, but do not preclude the presence or addition of one or more other features, integers, steps, operations, elements, and/or components.

A first direction (X direction), a second direction (Y direction), and a third direction (Z direction) described in one embodiment of the present invention may be directions orthogonal to each other.

Each of the first direction (X direction) and the second direction (Y direction) may be a direction parallel to the horizontal direction, and the third direction (Z direction) may be a direction parallel to the vertical direction. When the first direction (X direction) is parallel to a left-right direction, the second direction (Y direction) may be parallel to a front-rear direction. When the first direction (X direction) is parallel to the front-rear direction, the second direction (Y direction) may be parallel to the left-right direction.

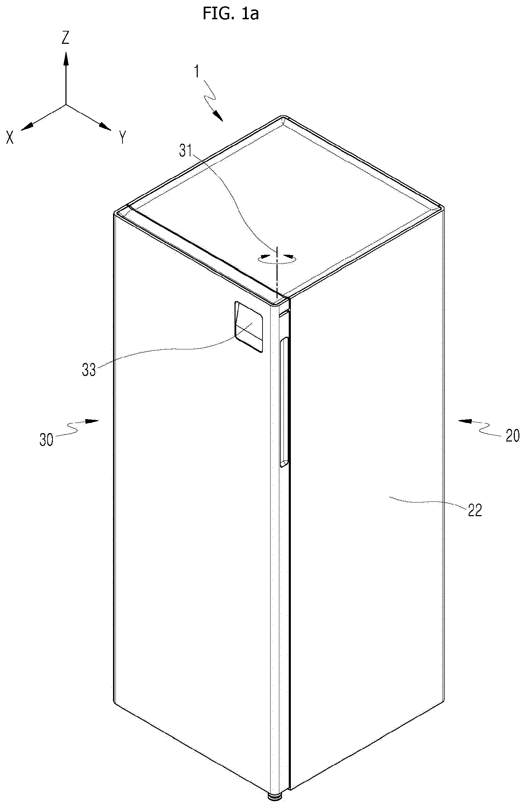

FIG. 1a is a perspective view illustrating a shoes care device according to one embodiment of the present invention.

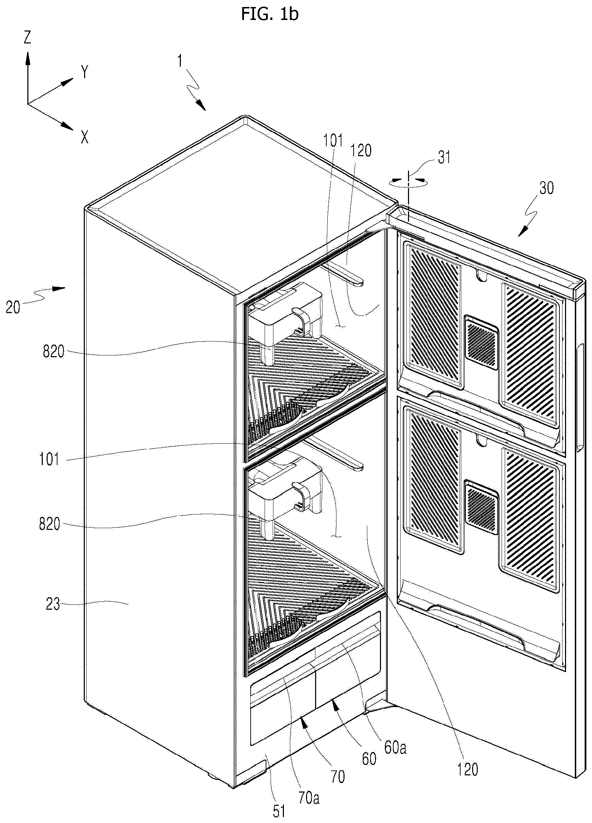

FIG. 1b is a perspective view of the shoes care device of FIG. 1a, when viewed from another direction, illustrating a state in which a door is opened.

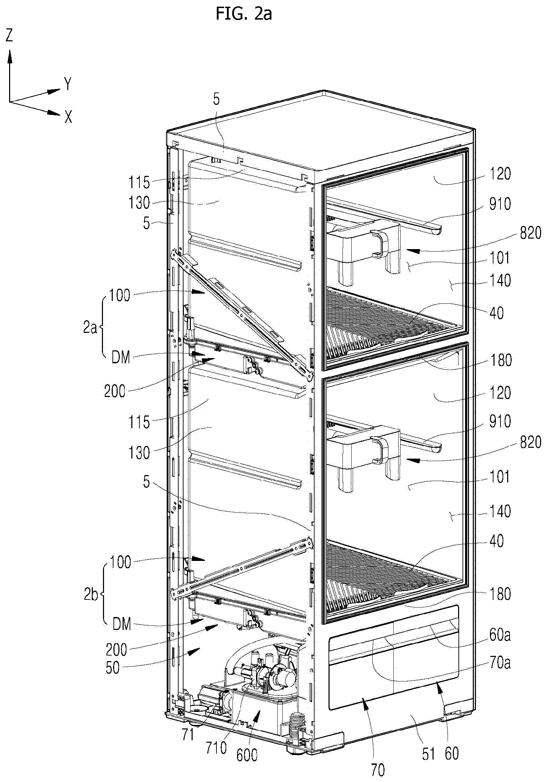

FIG. 2a is a perspective view illustrating a state in which a part of the door and an outer cabinet are removed from the shoes care device of FIG. 1b.

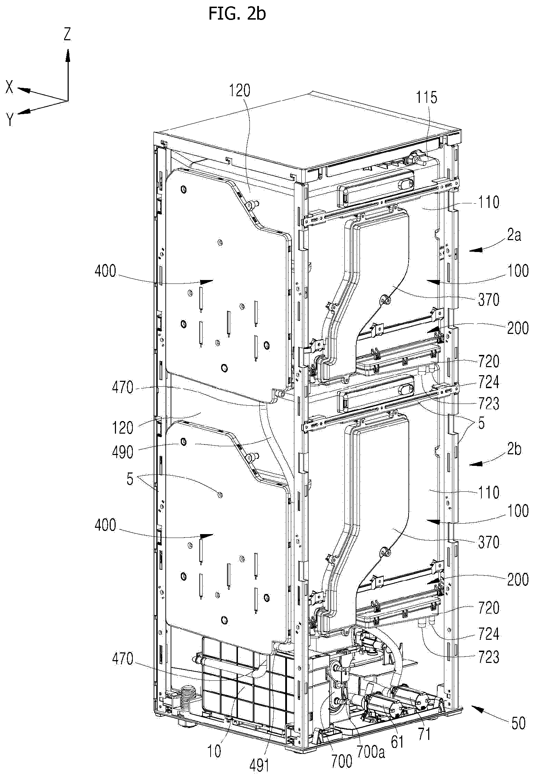

FIG. 2b is a perspective view illustrating a state in which the shoes care device illustrated in FIG. 2a is viewed from another direction.

A shoes care device 1 according to one embodiment of the present invention may include an outer cabinet 20, a door 30, an inner cabinet 100, and a machine room 50. The shoes care device 1 may include a main frame 5.

The outer cabinet 20 and the door 30 may form an overall appearance of the shoes care device 1. The outside of the shoes care device 1 may be formed in a hexahedral shape. That is, while the outer cabinet 20 and the door 30 are coupled to each other and the door 30 is closed, the appearance of the shoes care device 1 may be formed in a hexahedral shape. However, the shoes care device 1 according to one embodiment of the present invention is not limited to such a shape and may have various three-dimensional shapes.

When the door 30 forms the front of the shoes care device 1, the outer cabinet 20 may form an upper side surface, a left-side surface, a right-side surface, a rear surface, and bottom surfaces of the shoes care device 1.

The main frame 5 may form an overall framework of the shoes care device 1. The main frame 5 may have a hexahedral structure.

The outer cabinet 20 may be detachably fixed to the main frame 5.

The outer cabinet 20 may include an outer rear plate 21, a first outer side plate 22, and a second outer side plate 23.

The outer back plate 21, the first outer side plate 22, and the second outer side plate 23 may be formed integrally with each other, or the outer back plate 21, the first outer side plate 22, and the second outer side plate 23 may be individually formed.

The outer rear plate 21 forms a vertically erected wall surface. The outer rear plate 21 may form a surface orthogonal to the first direction (X direction). The outer rear plate 21 may form a rear wall surface in the first direction (X direction) on the outer cabinet 20. The outer rear plate 21 may form a rear surface in the first direction (X direction) in the shoes care device 1. The outer rear plate 21 may form an entire outer rear surface of the shoes care device 1.

The first outer side plate 22 and the second outer side plate 23 form vertically erected wall surfaces, respectively, and form opposing wall surfaces facing each other.

The first outer side plate 22 is disposed at any one side with respect to a reference plane RP that is parallel to the first direction (X direction) as a horizontal direction and is a vertical plane. The second outer side plate 23 is disposed on the opposite side of the first outer side plate 22 with respect to the reference plane RP. The first outer side plate 22 may form a left wall surface of the outer cabinet 20, and the second outer side plate 23 may form a right wall surface of the outer cabinet 20.

The outer cabinet 20 may be disposed outside the inner cabinet 100 and the machine room 50 to form an outer wall surface of the machine room 50. When a separate cabinet for the machine room 50 is not provided in the shoes care device 1, the outer cabinet 20 may form a wall separating the machine room 50 from the outside.

The door 30 is configured to open and close the inside of the shoes care device 1. The door 30 may form any one surface of the shoes care device 1. The door 30 may form a left side or a right side of the shoes care device 1, or may form a front side of the shoes care device 1.

In the shoes care device 1, the door 30 may be hinge-coupled.

In one embodiment, the door 30 may be hinge-coupled to the main frame 5. In another embodiment, the door 30 may be hinge-coupled to the outer cabinet 20, and in another embodiment, the door 30 may be hinge-coupled to the inner cabinet 100 and/or the machine room 50.

A hinge rotation axis 31 of the door 30 may be formed in the vertical direction. That is, in the shoes care device 1, the door 30 may be configured to be rotatable bidirectionally around a rotation axis 31 in the vertical direction.

In one embodiment, the shoes care device 1 may include one door 30. In another embodiment, the shoes care device 1 may include two or more doors.

When two doors are provided in the shoes care device 1, each door may be individually rotated around each rotation axis.

The first direction (X direction) described in one embodiment of the present invention may be parallel to or substantially parallel to the horizontal direction.

In one embodiment, the first direction (X direction) may be a direction from the rear of the shoes care device 1 to the front.

Hereinafter, except for a particularly limited case, it will be described that the door 30 is formed in a front side of the shoes care device 1. That is, a surface on which the door 30 is formed in the shoes care device 1 is described as a front surface of the shoes care device 1.

Furthermore, hereinafter, except for a particularly limited case, it will be described that the first direction (X direction) is parallel to the front-rear direction, the second direction (Y direction) is parallel to the left-right direction, and the third direction (Z direction) is parallel to the vertical direction.

The inner cabinet 100 and the machine room 50 may be provided inside the outer cabinet 20.

The inner cabinet 100 may be formed in a box shape, and a predetermined space may be formed therein. The space inside the inner cabinet 100 forms an accommodation space 101, and shoes S may be accommodated in the accommodation space 101.

A plurality of shoes S may be disposed together in the accommodation space 101 of one inner cabinet 100.

The inner cabinet 100 may be fixed to the main frame 5.

The inner cabinet 100 has a predetermined size along the first direction (X direction), the second direction (Y direction), and the third direction (Z direction).

The inner cabinet 100 is formed in the shape of a box opened to any one side. The inner cabinet 100 may be formed in a form opened to the front side of the shoes care device 1. The inner cabinet 100 may be configured to include a main opening 140. The main opening 140 may be provided to open the front side of the inner cabinet 100 in the first direction (X direction). The shoes may be disposed inside the inner cabinet 100 or withdrawn from the inner cabinet 100 through the main opening 140.

The main opening 140 of the inner cabinet 100 may be closed or opened by the door 30.

The inner cabinet 100 may include an inner rear plate 110, a first inner side plate 120, a second inner side plate 130, and an inner upper plate 115.

The inner rear plate 110, the first inner side plate 120, the second inner side plate 130, and the inner upper plate 115 may be formed integrally with each other. The inner cabinet 100 may be made of a single material and be formed by injection molding.

The inner rear plate 110 forms a vertically erected wall surface. The inner rear plate 110 may form a surface orthogonal to the first direction (X direction). The inner rear plate 110 may form a rear wall surface of the inner cabinet 100 in the first direction (X direction). The inner rear plate 110 may be formed parallel to the outer rear plate 21.

The first inner side plate 120 and the second inner side plate 130 form vertically erected wall surfaces, respectively, and form opposing wall surfaces facing each other.

The first inner side plate 120 is dispose on either side with respect to the reference plane RP that is parallel to the first direction (X direction) as the horizontal direction and is a vertical surface. The second inner side plate 130 is disposed on the opposite side of the first inner side plate 120 with respect to the reference plane RP. The first inner side plate 120 forms a left wall surface of the inner cabinet 100, and the second inner side plate 130 forms a right wall surface of the inner cabinet 100.

The first inner side plate 120 may be formed parallel to the first outer side plate 22, and the second inner side plate 130 may be formed parallel to the second outer side plate 23.

In one embodiment of the present invention, the inner cabinet 100 may have the form where a lower part thereof is opened. Accordingly, the inner cabinet 100 includes a lower opening 150 formed by opening the lower part thereof. When the inner cabinet 100 is formed in a hexahedral shape on the whole, the lower opening 150 may be formed large to form all or most of a lower surface of the inner cabinet 100 (see FIGS. 53 and 54).

However, the shoes care device 1 according to one embodiment of the present invention is not used in a state in which an entire lower part of the inner cabinet 100 is opened, but is used in a state in which the inner cabinet 100 and a module housing 200 are coupled to each other so that the lower opening 150 of the inner cabinet 100 is shielded by the module housing 200. That is, the shoes care device 1 is used so that an upper surface of the module housing 200 forms a bottom surface of the accommodation space 101 of the inner cabinet 100. A further explanation thereof will be described below.

A main shelf 40 may be provided in the inside 101 of the inner cabinet 100. The main shelf 40 may be formed such that the shoes S are settled on an upper surface thereof.

The main shelf 40 may be formed in the form of a plate with a predetermined area, or may be formed in the form of a grill where multiple bars are spaced apart from each other.

One main shelf 40 may be provided, or a plurality of main shelves 40 may be provided.

The main shelf 40 may have a substantially flat plate shape and be disposed on a bottom surface of the inner cabinet 100. The main shelf 40 is settled on an upper side of a module cover 202 of the inner cabinet 100. The main shelf 40 may be disposed on the upper side of the module cover 202 of the inner cabinet 100 in a stacked form.

The main shelf 40 is detachable from the inner cabinet 100, and when the main shelf 40 is withdrawn from the inner cabinet 100, the upper surface of the module housing 200 is exposed.

The main shelf 40 may have a rectangular shape when viewed in a plan view. The size of the main shelf 40 may be a size corresponding to the bottom of the accommodation space 101 of the inner cabinet 100. That is, when the main shelf 40 is disposed inside the inner cabinet 100, the main shelf 40 may form all or most of the bottom of the accommodation space 101 of the inner cabinet 100.

In one embodiment, the machine room 50 may be provided in a lower side of the inner cabinet 100. In the machine room 50, some of the components that constitutes the shoes care device 1 may be accommodated, and, in this case, the components that are accommodated in the machine room 50 may be fixed to a main frame 5 or to the inner cabinet 100 or the outer cabinet 20.

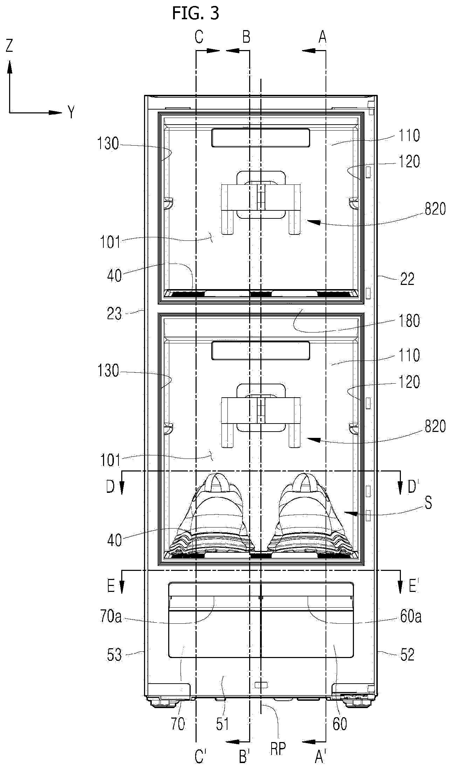

FIG. 3 is a front view illustrating a state in which a door is removed from the shoes care device illustrated in FIG. 1b. FIG. 3 illustrates shoes accommodated in an inner cabinet together.

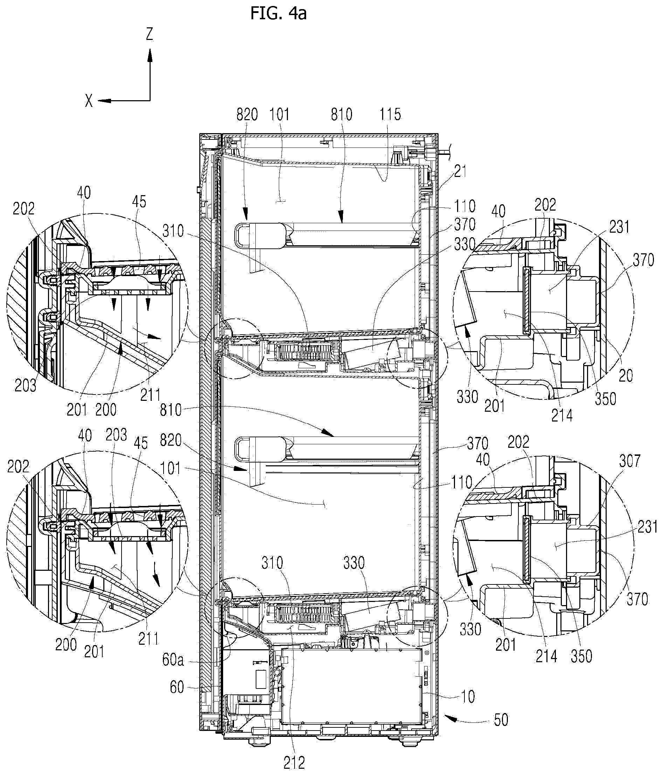

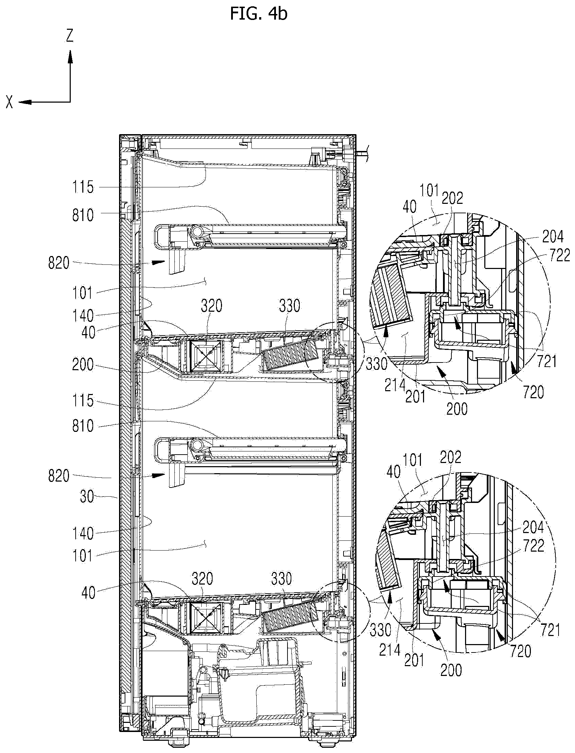

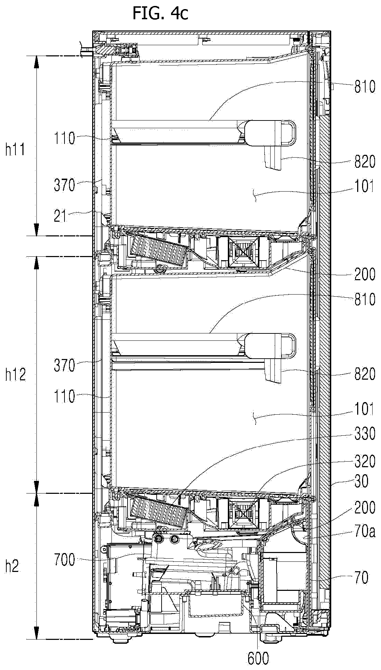

FIG. 4a is a cross-sectional view taken along line A-A' of the shoes care device illustrated in FIG. 3, FIG. 4b is a cross-sectional view taken along line B-B' of the shoes care device illustrated in FIG. 3, and FIG. 4c is a cross-sectional view taken along line C-C' of the shoes care device illustrated in FIG. 3. FIGS. 4a to 4c illustrate a form where a door is included in the shoes care device, and do not illustrate shoes.

The shoes care device 1 according to one embodiment of the present invention includes management devices 2a and 2b. In the shoes care device 1, a plurality of management devices 2a and 2b may be provided. In one embodiment, the shoes care device 1 may include a first management device 2a and a second management device 2b. That is, the shoes care device 1 may include two separate management devices 2a and 2b.

The 'management device' described in the present invention may refer to a 'first management device 2a' and a 'second management device 2b', respectively, except for a particularly limited case.

The management devices 2a and 2b include the above-described inner cabinet 100.

In one embodiment of the present invention, since the inner cabinet 100 of the first management device 2a and the inner cabinet 100 of the second management device 2b may be distinguished from each other, the inner cabinet 100a of the first management device 2a may refer to a first inner cabinet 100a, and inner cabinet 100a of the second management device 100b may refer to a second inner cabinet 100b.

It may be understood that the 'inner cabinet 100' described in one embodiment of the present invention refers to each of the 'first inner cabinet 100a' and the 'second inner cabinet 100b', except for a particularly limited case.

When the door 30 is closed in the shoes care device 1, the door 30 closes the main opening 140 of the first management device 2a and also closes the main opening 140 of the second management device 2b. That is, the door 30 may simultaneously seal the accommodation space 101 of the inner cabinet 100 of the first management device 2a and the accommodation space 101 of the inner cabinet 100 of the second management device 2b. In this case, the accommodation space 101 of the inner cabinet 100 of the first management device 2a and the accommodation space 101 of the inner cabinet 100 of the second management device 2b may be configured such that they do not communicate with each other.

As described above, in one embodiment of the present invention, the accommodation space 101 of the inner cabinet 100 of the first management device 2a and the accommodation space 101 of the inner cabinet 100 of the second management device 2b may form independent spaces, and may form blocked spaces (not communicating with each other). Accordingly, the temperature and humidity of the accommodation space 101 of the first management device 2a and the temperature and humidity of the accommodation space 101 of the second management device 2b may be controlled differently from each other.

The management devices 2a and 2b may be configured to include a connection path F10. The management devices 2a and 2b may be configured to include a blowing part 310 and a dehumidifying part 330. The management devices 2a and 2b may include a outlet 203 and a nozzle 820.

The management devices 2a and 2b may include the module housing 200 forming a connection path F10.

The management devices 2a and 2b may include a regeneration path F20. The management devices 2a and 2b may include a heating part 320.

The management devices 2a and 2b may include a conversion flow path F10a.

The management devices 2a and 2b may include a damper 350.

The shoes care device 1 may include a steam generator 700 and a steam valve 710.

The shoes care device 1 may include a sump 600, a water supply tank 60, and a drain tank 70.

Since all or part of the outer cabinet 20 may be spaced apart from the inner cabinet 100, a predetermined gap may be formed between the inner cabinet 100 and the outer cabinet 20.

In a space between the inner cabinet 100 and the outer cabinet 20, components constituting the shoes care device 1 may be provided, and various flow paths constituting the shoes care device 1 may be provided. In one embodiment of the present invention, part of the connection path F10 may be provided between the inner cabinet 100 and the outer cabinet 20, and part of the regeneration path F20 may be provided between the inner cabinet 100 and the outer cabinet 20.

A dry air duct 370 forming the connection path F10 may be provided between the outer rear plate 21 and the inner rear plate 110. Furthermore, a condenser 400 forming the regeneration path F20 may be provided between the outer rear plate 21 and the inner rear plate 110.

The connection path F10 forms a flow path of a fluid.

The connection path F10 forms a passage through which air and/or condensed water inside the shoes care device 1 moves.

The dehumidifying part 330 is disposed inside the connection path F10 and includes a dehumidifying material. The dehumidifying part 330 may be entirely formed of the dehumidifying material, or a part thereof may be formed of the dehumidifying material. A further explanation of the dehumidifying part 330 will be described below.

According to one embodiment of the present invention, the shoes care device 1 has an air circulation structure in which the air inside the inner cabinet 100 in which the shoes are disposed is sucked into the connection path F10 to dehumidify the air using a dehumidifying material 331 and the dehumidified air may be supplied back into the inner cabinet 100.

The connection path F10 may be used as a means to achieve such an air circulation structure in the shoes care device 1. All or part of the connection path F10 may be formed of a pipe, a hose, a tube, a duct, a housing, or a combination thereof.

The module housing 200 according to one embodiment of the present invention forms part of the connection path F10.

The outlet 203 is formed in the module housing 200. The outlet 203 communicates with the accommodation space 101 of the inner cabinet 100, and forms an inlet of the module housing 200 through which the air of the accommodation space 101 of the inner cabinet 100 is suctioned into the module housing 200. The outlet 203 may be disposed below the main shelf 40. In this case, the main shelf 40 may be formed so as not to block the air in the accommodation space 101 from being sucked into the outlet 203. To this end, when the main shelf 40 is formed in a plate shape, a plurality of holes 45 penetrating in the vertical direction may be formed in the main shelf 40 so as to move the air.

In the shoes care device 1, the inner cabinet 100 and the machine room 50 may form a space separated from each other. Furthermore, the module housing 200 that is part of the management devices 2a and 2b may be provided between the inner cabinet 100 and the machine room 50.

The inner cabinet 100, the module housing 200, and the machine room 50 are provided inside the shoes care device 1 according to one embodiment of the present invention.

The inner cabinet 100, the module housing 200, and the machine room 50 may be continuously arranged from the upper side to the lower side. When the shoes care device 1 according to one embodiment of the present disclosure includes the first management device 2a and the second management device 2b, the first management device 2a may be disposed above the second management device 2b. That is, the first management device 2a, the second management device 2b, and the machine room 50 may be continuously arranged from the upper side to the lower side.

Since the first management device 2a and the second management device 2b include the inner cabinet 100 and the module housing 200, respectively, they may be continuously arranged in an order of the inner cabinet 100 of the first management device 2a, the module housing 200 of the first management device 2a, the inner cabinet 100 of the second management device 2b, the module housing 200 of the second management device 2b, the machine room 50 from the upper side to the lower side.

The inner cabinet 100 may form a space that mainly accommodates an article (shoes S) to be managed, and the module housing 200 and the machine room 50 may form a space that mainly accommodates components for an operation of the shoes care device 1.

In the shoes care device 1 according to one embodiment of the present invention, the blowing part 310, the dehumidifying part 330 (and the dehumidifying material 331), and the heating part 320 may be accommodated inside the module housing 200.

In addition, the machine room 50 may be configured to accommodate a controller 10, the sump 600, the steam generator 700, and the steam valve 710 therein. Furthermore, the machine room 50 may be configured to accommodate the water supply tank 60 and the drain tank 70.

Among the components constituting the management devices 2a and 2b, components not included in the module housing 200 may be fixedly coupled to the inner cabinet 100 and the outside of the module housing 200 or may be fixedly coupled to the main frame 5.

Components coupled to or accommodated in the machine room 50 may be fixedly coupled to the machine room 50.

The machine room 50 may include a first wall 51.

A first wall 51 forms one wall surface of the machine room 50. The first wall 51 may be erected in the vertical direction, or may be erected in the substantially vertical direction. In one embodiment, the first wall 51 may form a wall surface orthogonal to or inclined with the first direction (X direction).

The first wall 51 may form a front wall surface of the machine room 50, a left wall of the machine room 50, or a right wall of the machine room 50.

The machine room 50 may include a second wall 52 and a third wall 53. The second wall 52 and the third wall 53 form opposite wall surfaces facing each other in the machine room 50. The second wall 52 and the third wall 53 may be erected in the vertical direction, or may be erected in the substantially vertical direction.

When the first wall 51 forms a front wall of the machine room 50, the second wall 52 may form a left wall of the machine room 50, and the third wall 53 may form a right wall of the machine room 50.

The first wall 51 may be formed integrally with the inner cabinet 100, and the second wall 52 and the third wall 53 may be formed integrally with the outer cabinet 20, respectively.

Each of the water supply tank 60 and the drain tank 70 may be formed in the form of a container for accommodating water.

The water supply tank 60 may be configured to store water supplied into the shoes care device 1 inside. The water supply tank 60 may be configured to store water supplied into the steam generator 700 inside.

In order to supply water from the water supply tank 60 into the shoes care device 1, a water pump 61 may be connected to the water supply tank 60. The water supply tank 60 for moving water and the first water pump 61 may be connected by a pipe, a hose, etc.

The drain tank 70 may be configured to store water discharged from the shoes care device 1 inside. The drain tank 70 may store water condensed inside the shoes care device 1. The drain tank 70 may be configured to store water drained from the sump 600.

In order to discharge water to the drain tank 70, a water pump 71 (a second water pump) may be connected to the drain tank 70. The drain pipe 70 for moving water and the second water pump 71 may be connected by a pipe, a hose, etc.

The water supply tank 60 and the drain tank 70 may be coupled to the machine room 50 to get exposed from the outside of one wall surface of the machine room 50.

The water supply tank 60 and the drain tank 70 may be disposed in front of the machine room 50.

The water supply tank 60 and the drain tank 70 may form one wall surface of the machine room 50 along with the first wall 51. When the first wall 51 forms a front surface of the machine room 50, the water supply tank 60 and the drain tank 70 may be exposed from a front side of the machine room 50, and may be coupled to the machine room 50 to get exposed from the outside of the first wall 51.

As the water supply tank 60 and the drain tank 70 are exposed to the outside of the first wall 51, a user may inject water into the water supply tank 60 or discharge water from the drain tank 70.

The water supply tank 60 and the drain tank 70 may be configured to be detachable from the machine room 50. The water supply tank 60 and the drain tank 70 may be detached from the first wall 51. In order to facilitate the attachment and detachment of the water supply tank 60 and the drain tank 70, a handle 60a of the water supply tank 60 may be formed on an outer surface of the water supply tank 60, and a handle 70a of the drain tank 70 may be formed on an outer surface of the drain tank 70.

Each of the water supply tank 60 and the drain tank 70 may be configured to be separated from the machine room 50 in an outer direction of the first wall 51.

The controller 10 may be configured to control operations of each component in connection with each component constituting the shoes care device 1.

In order to control the controller 10, the shoes care device 1 may be provided with a storage medium in which an application program is stored, and the controller 10 may be configured to control the shoes care device 1 by driving an application program according to information input to the shoes care device 1 and information output from the shoes care device 1.

The controller 10 may control the first management device 2a and the second management device 2b constituting the shoes care device 1 to operate individually. The controller 10 may control the first management device 2a and the second management device 2b to operate in different states, and may control shoes (e.g., sneakers) in the first management device 2a and shoes (e.g., heels) in the second management device 2b to be managed under different conditions. Furthermore, the controller 10 may control the first management device 2a and the second management device 2b to interwork with each other.

The door 30 may be disposed on either the inner cabinet 100 or the machine room 50 on the same side as the first wall 51. When the door 30 forms the front surface of the shoes care device 1, the first wall 51 forms the front surface of the machine room 50, and the door 30 is disposed directly outside the first wall 51.

In one embodiment of the present invention, the door 30 may be configured to open and close the inner cabinet 100 and further expose or shield the front surface of the machine room 50.

The door 30 may be configured to expose or shield the inner cabinet 100, the water supply tank 60, and the drain tank 70.

As described above, in the shoes care device 1, the door 30, the water supply tank 60, and the drain tank 70 are formed on the same side, and when the door 30 is opened, the water supply tank 60 and the drain tank 70 may be exposed and separated from the shoes care device 1.

In the arrangement explained above, even if left and right sides and a rear side of the shoes care device 1 are blocked by other goods or structures, the door 30 may be opened on a front side of the shoes care device 1, and the water supply tank 60 and the drain tank 70 can be separated from or coupled again to the shoes care device 1.

A control panel 33 for controlling the shoes care device 1 is provided on an outer side of the door 30. The control panel 33 may be formed of a touch screen. A control unit (controller 10) is provided in the inner space of the door 30 to control each component of the shoes care device 1 in connection with the control panel 33. The controller 10 may be provided inside the machine room 50.

As illustrated in FIGS. 1a and 1b, in one embodiment, the door 30 may be configured to simultaneously expose or shield the inner cabinet 100 and the machine room 50.

In another embodiment, the door 30 may be configured to open and close only the inner cabinet 100. In this case, the machine room 50 may not be shielded by the door 30. Furthermore, in this case, the shoes care device 1 according to one embodiment of the present invention may be further provided with a dedicated door of the machine room 50 to open and close the machine room 50 separately from the door 30.

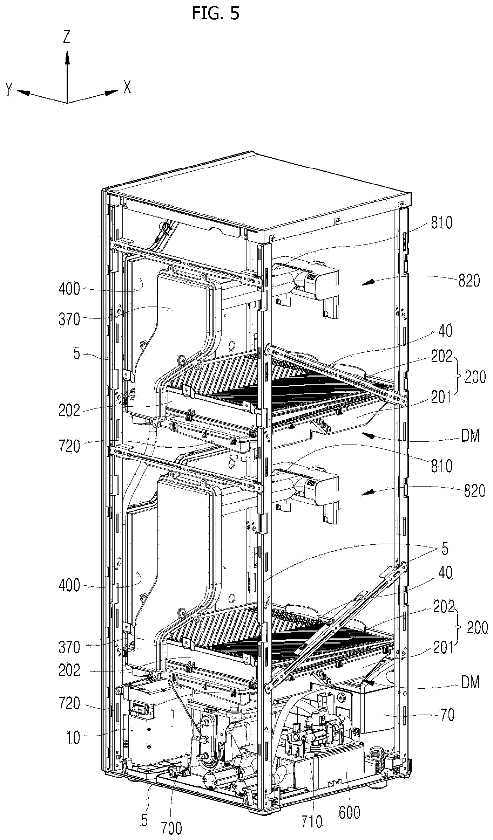

FIG. 5 is a perspective view illustrating a state in which the door 30, the outer cabinet 20, and the inner cabinet 100 are removed from the shoes care device 1 according to one embodiment of the present invention.

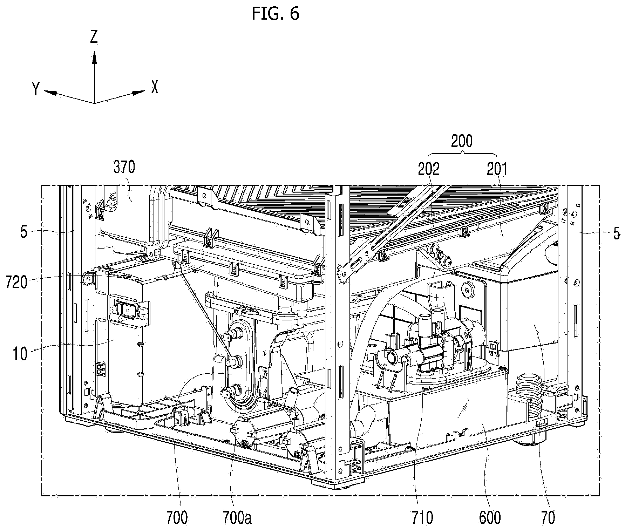

FIG. 6 is a perspective view illustrating a machine room part of the shoes care device 1 illustrated in FIG. 5.

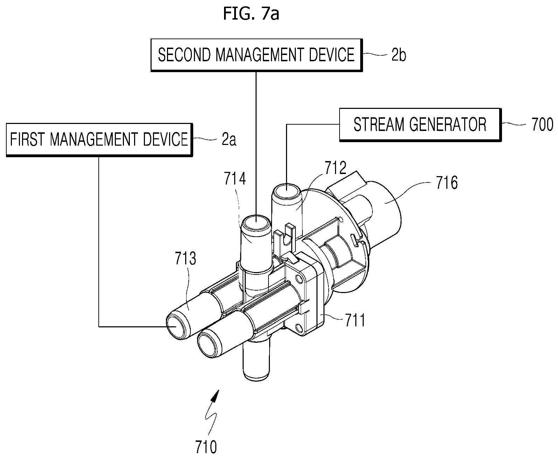

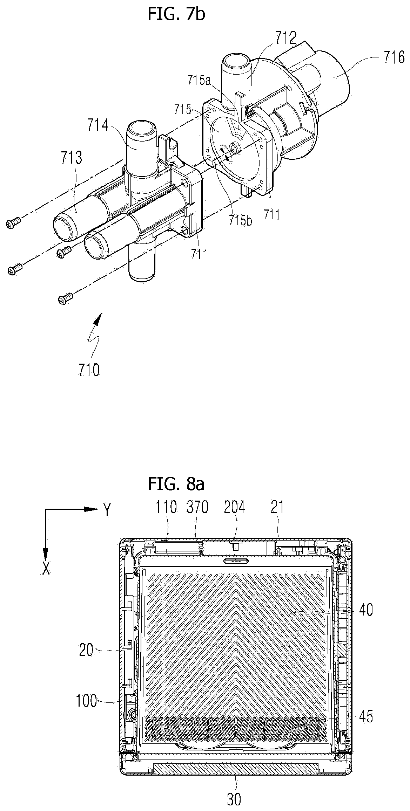

FIG. 7a is a view illustrating the steam valve 710 according to one embodiment of the present invention, and illustrating a connection relationship considering the movement of steam. FIG. 7b is an exploded perspective view illustrating the steam valve illustrated in FIG. 7a.

The shoes care device 1 is provided with the steam generator 700 as a device configured so as to generate moisture inside the inner cabinet 100. The steam generator 700 may be provided inside the machine room 50. The steam generator 700 is configured to generate steam and selectively supply moisture and steam to the inside of the inner cabinet 100.

The shoes care device 1 according to one embodiment may be provided with one steam generator 700, and the shoes care device 1 according to another embodiment may be provided with two or more steam generators 700.

When the shoes care device 1 is provided with one steam generator 700, the steam generator 700 may be configured to supply steam into the inner cabinet 100 of the first management device 2a and/or into the inner cabinet 100 of the second management device 2b.

When the shoes care device 1, one steam generator 700 may be provided with two or more steam generators 700, one of the steam generators 700 may supply steam to the inner cabinet 100 of the first management device 2a, and the other steam generator 700 may supply steam to the inner cabinet 100 of the second management device 2b.

Moist air formed by the steam generator 700 ('air' described in one embodiment of the present invention may be 'air including moisture') is supplied toward the accommodation space 101 of the inner cabinet 100, and moisture may be circulated in the accommodation space 101 of the inner cabinet 100, and accordingly, the moisture may be supplied to the shoes S.

The shoes care device 1 according to one embodiment of the present invention may be a refresher device that refreshes the shoes.

Here, refreshing may refer to a process of removing contaminants, deodorizing, sanitizing, preventing static electricity, or warming by supplying air, heated air, water, mist, steam, etc. to the shoes.

The steam generator 700 may supply steam to the accommodation space 101 of the inner cabinet 100 in which the shoes S are accommodated, and may perform steam treatment on the shoes, which is further meant to exert a refreshing effect due to swelling of shoes materials as well as sterilization by high-temperature steam.

The steam generator 700 is provided with a separate heater 700a that heats an inner space and water in the inner space and is configured to heat water to generate steam and supply the heated water to the accommodation space 101 of the inner cabinet 100.

An external faucet, etc., as a water supply source for supplying water to the steam generator 700, may be used, or a container-type water supply tank provided on one side of the machine room 50 may be used. The steam generator 700 may generate steam by receiving water from the water supply tank 60.

The water supply tank 60 for the movement of water and the steam generator 700 may be connected by a pipe, a hose, etc.

The steam generator 700 for the movement of steam and the inner cabinet 100 may be connected by a pipe, a hose, etc. In the shoes care device 1 according to one embodiment of the present invention, as described below, the steam generated by the steam generator 700 may be supplied into the inner cabinet 100 after passing through the steam valve 710 and a steam separator 720. In this case, in order to move the steam, the steam generator 700 and the steam valve 710 may be connected by a pipe, a hose, etc., and the steam valve 710 and the steam separator 720 may also be connected by a pipe, a hose, etc.

The steam valve 710 may be disposed adjacent to the steam generator 700, and the steam valve 710 may be provided in the machine room 50. The steam generator 700 and the steam valve 710 may be provided below the second management device 2b.

The steam valve 710 is configured to selectively communicate with each of the accommodation space 101 of the first management device 2a and the accommodation space 101 of the second management device 2b, respectively.

When the steam of the steam generator 700 is supplied to the accommodation space 101 of the first management device 2a and/or the accommodation space 101 of the second management device 2b, the steam valve 710 operates to control whether the stream is supplied or not, and an operation of the stream 710 is driven by the controller 10.

The steam valve 710 includes a valve housing 711, a valve inlet 712, a first valve outlet 713, a second valve outlet 714, a valve disk 715, and a valve motor 716. In the steam valve 710 illustrated in FIGS. 7a and 7b, the other outlets except the valve inlet 712, the first valve outlet 713, and the second valve outlet 714 may be blocked by a stopper and deactivated.

The valve housing 711 forms a body of the steam valve 710, and has a predetermined inner space formed inside.

The valve inlet 712 may have a tubular shape and be coupled to the valve housing 711 to communicate with the inner space of the valve housing 711. The valve inlet 712 is a part connected to the steam generator 700, and steam may flow into the steam valve 710 (inside the valve housing 711) through the valve inlet 712.

The first valve outlet 713 and the second valve outlet 714 may be formed in a tubular shape and be coupled to the valve housing 711 to communicate with the inner space of the valve housing 711.

The first valve outlet 713 and the second valve outlet 714 are outlets through which steam is discharged from the steam valve 710. The first valve outlet 713 is connected to the accommodation space 101 of the first management device 2a, and the second valve outlet 714 is connected to the accommodation space 101 of the second management device 2b.

The valve disk 715 is provided inside the steam valve 710 (inside the valve housing 711) and is configured to open and close a flow path inside the steam valve 710. The valve disk 715 may be configured to selectively open and close a flow path of the first valve outlet 713 and a flow path of the second valve outlet 714.

The valve disk 715 is disposed between the valve inlet 712 and the first valve outlet 713, or between the valve inlet 712 and the second valve outlet 714, inside the valve housing 711. The valve disk 715 allows the valve inlet 712 and the first valve outlet 713 to communicate with each other or block the communication therewith, and also allows the valve inlet 712 and the second valve outlet 714 to communicate with each other or block the communication therewith.

In one embodiment of the present invention, the valve disk 715 may be formed in a circular plate shape and may include a valve hole 715a. The valve hole 715a is a hole penetrating the valve disk 715. The valve disk 715 may be rotatably coupled to the valve housing 711 around a valve rotation axis 715b, and the valve hole 715a may be formed eccentrically from the valve rotation axis 715b.

The valve motor 716 is coupled to the valve housing 711, and the valve motor 716 is coupled to the rotation axis 715b of the valve disk 715 to rotate the valve disk 715.

The controller 10 may control the steam valve 710 by controlling an operation of the valve motor 716.

The valve disk 715 rotates by the operation of the valve motor 716, and the valve disk 715 opens or closes a flow path inside the steam valve 710 (inside the valve housing 711) depending on the degree of rotation of the valve disk 715.

In one embodiment, depending on the degree of rotation of the valve disk 715, when the valve inlet 712 and the first valve outlet 713 communicate with each other through the valve hole 715a, the valve inlet 712 and the second valve outlet 714 are blocked from communicating with each other by the valve disk 715, or when the valve inlet 712 and the second valve outlet 714 communicate with each other through the valve hole 715a, the valve outlet 712 and the first valve outlet 713 may be blocked from communicating with each other by the valve disk 715.

In another embodiment, depending on the degree of rotation of the valve disk 715, the valve inlet 712 may communicate with both the first valve outlet 713 and the second valve outlet 714, or the valve inlet 712 may be blocked from communicating with both the first valve outlet 713 and the second valve outlet 714.

In the shoes care device 1 according to one embodiment of the present invention, the valve disk 715 may close a flow path of the second valve outlet 714 while opening a flow path of the first valve outlet 713, and may also close the flow path of the first valve outlet 713 and open the flow path of the second valve outlet 714.

The steam valve 710 made as described above may operate such that only the valve inlet 712 and the first valve outlet 713 communicate with each other, or only the valve inlet 712 and the second valve outlet 714 communicate with each other.

In the arrangement explained above, all the steam generated by the steam generator 700 may be supplied to the accommodation space 101 of the first management device 2a, or may be supplied to the accommodation space 101 of the second management device 2b. In this case, the steam generated by the steam generator 700 may be supplied to the accommodation space 101 of the first management device 2a or the accommodation space 101 of the second management device 2b without a pressure drop, and even when only one steam generator 700 is provided in the shoes care device 1, the steam may be sufficiently and stably supplied to the accommodation spaces 101 of each of the two inner cabinets 100.

In one embodiment of the present invention, the controller 10 may control the stream valve 710 such that when the heating part 320 of the first management device 2a is turned off (when a heater 321 of the heating part 320 is turned off), the valve disk 715 closes or opens the first valve outlet 713, and when the heating part 320 of the first management device 2a is turned on (when the heater 321 of the heating part 320 is turned on), the valve disk 715 closes the first valve outlet 713.

In addition, the controller 10 may control the stream valve 170 such that when the heating part 320 of the second management device 2b is turned off (when the heater 321 of the heating part 320 is turned off), the valve disk 715 closes or opens the second valve outlet 714, and when the heating part 320 of the second management device 2b is turned on (when the heater 321 of the heating part 320 is turned on), the valve disk 715 closes the second valve outlet 714.

In the shoes care device 1 according to one embodiment of the present invention, by controlling the steam valve 710 by the controller 10, the steam generated in the steam generator 700 may be selectively or simultaneously supplied to the accommodation space 101 of the first management device 2a and the accommodation space 101 of the second management device 2b, and whether the steam is supplied or not may be controlled according to a use state of the shoes care device 1.

FIG. 8a is a cross-sectional view taken along line D-D' of the shoes care device 1 illustrated in FIG. 3.

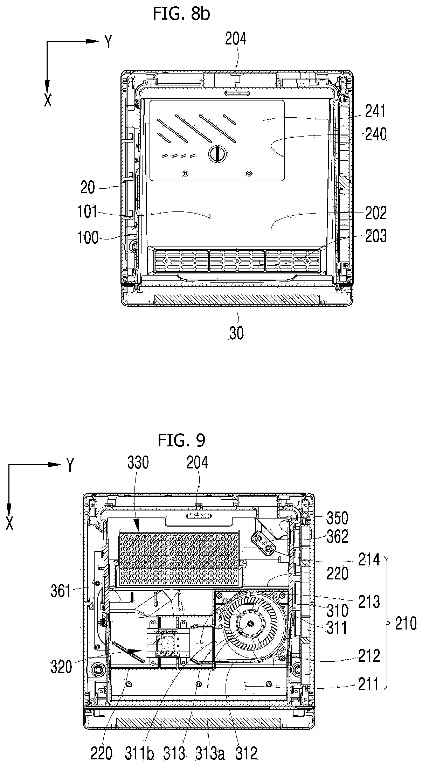

FIG. 8b is a view illustrating a state in which the main shelf 40 is removed from the shoes care device 1 illustrated in FIG. 8a.

FIG. 9 is a cross-sectional view taken along line E-E' of the shoes care device 1illustrated in FIG. 3.

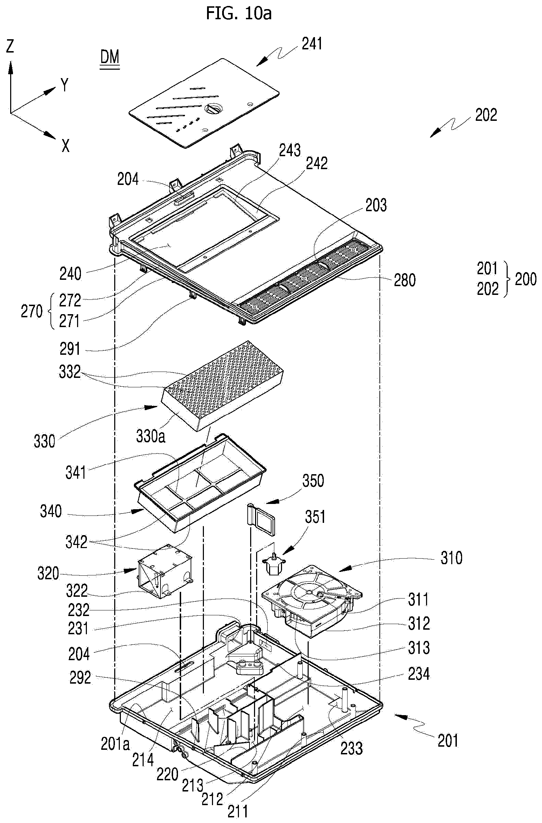

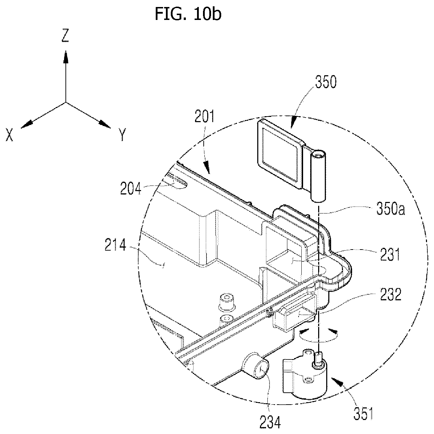

FIG. 10a is an exploded perspective view illustrating a drying module in the shoes care device 1 according to one embodiment of the present invention. FIG. 10b is an exploded perspective view illustrating a partial configuration of the drying module at a part to which the damper 350 is coupled.

In the shoes care device 1 according to one embodiment of the present invention, the dehumidifying part 330 may be used as a means that dehumidifies air.

As described above, the dehumidifying part 330 may be provided inside the module housing 200.

The dehumidifying part 330 is configured to have a predetermined volume. The dehumidifying part 330 may be configured to be porous by itself. A plurality of pores may be formed over an entire volume of the dehumidifying part 330, and air may move by penetrating the dehumidifying part 330 through such pores.

When the dehumidifying part 330 is composed of a combination of a plurality of dehumidifying materials, the plurality of dehumidifying materials may be fixed to each other by a separate fixing means, or may be fixed to each other by adhesion.

The dehumidifying part 330 may be formed of dehumidifying materials, and may be configured to include the dehumidifying materials.

The dehumidifying material 331 according to one embodiment of the present invention is configured to include a material capable of reducing humidity by absorbing moisture in the air. The dehumidifying material 331 may be formed of various materials or a combination of the materials within the range of absorbing or adhering the moisture in the air, and may be formed in various shapes and structures.

The dehumidifying material 331 according to one embodiment of the present invention may be referred to as a desiccant or an adsorbent.

The dehumidifying material 331 according to one embodiment of the present invention may be formed of a microporous material. The dehumidifying material 331 according to one embodiment of the present invention may include silica gel, activated carbon, activated alumina (AL2O3), diatomaceous earth, etc.

Specifically, the dehumidifying material 331 according to one embodiment of the present invention may be formed of zeolite or may be configured to include zeolite.

The zeolite is a natural and synthetic silicate mineral in which tunnels or open channels having a size of approximately 3 to 10 angstroms (Å) are regularly arranged, and may function as dehumidification by adsorbing the moisture in the air.

When the zeolite is heated, moisture adsorbed on the zeolite may be separated into a large amount of steam. According to the characteristics of the zeolite, the zeolite may be regenerated in a state capable of not only performing the dehumidification function to remove the moisture from the air but also performing the dehumidification function by heating the zeolite and separating the moisture adsorbed to the zeolite.

The zeolite may be formed in the form of small grains (or stones) having the size (diameter) of several micrometers to several tens of micrometers, and the dehumidifying material 331 described in one embodiment of the present invention may refer to a combination of the grains (or stones). Each of the grains (or stones) may be agglomerated or combined with each other to form a single structure.

In another embodiment, the dehumidifying part 330 may include a dehumidifying body 330a and the dehumidifying material 331.

The dehumidifying body 330a may be formed to have a predetermined volume. In one embodiment, the dehumidifying body 330a may be formed in a substantially hexahedral shape.

In order to allow air to move through the dehumidifying body 330a, the dehumidifying body 330a may be provided with a plurality of dehumidification through holes 332 penetrated in one direction. A cross section of the dehumidifying through hole 332 may be formed in a circular shape, a polygonal shape, etc. The dehumidifying through hole 332 may have a hexagonal cross section.

In the dehumidifying body 330a, the dehumidifying through hole 332 may all have the same shape and size, or may have different shapes and sizes.

The dehumidifying body 330a may be formed of or include materials such as synthetic resin, metal, ceramic, etc. The dehumidifying body 330a may be formed of a combination of fibers, and may be formed of a nonwoven fabric, etc.

The dehumidifying material 331 may be coated on the dehumidifying body 330a. The dehumidifying material 331 may be coated on the outside and inside of the dehumidifying body 330a. Specifically, the dehumidifying material 331 may be coated on a surface in which the dehumidifying through hole 332 is formed.

When the dehumidifying body 330a is formed of a combination of fibers, the zeolite may be first coated on each fiber as the dehumidifying material 331, and the zeolite-coated fiber may be processed to form the dehumidifying body 330a and simultaneously form the dehumidifying part 330.

Regarding the coating of the zeolite, a manufacturing method of a zeolite coated ceramic paper (Korean Patent No. 10-1004826) is known, and Korean Patent No. 10-1173213 and Korean Patent No. 10-0941521 also describes a method of coating zeolite on a surface of a material. The dehumidifying part 330 according to one embodiment of the present invention may be formed by coating the gelled zeolite precursor on the dehumidifying body 330a or materials constituting the dehumidifying body 330a and then performing heat treatment when the dehumidifying material 331 is formed of the zeolite.

In one embodiment of the present invention, the coating of the dehumidifying material 331 (zeolite) may be performed by using various known or possible methods, and is not limited to a certain manufacturing method related to the coating of the dehumidifying material 331.

The blowing part 310 is provided inside the connection path F10. A blowing fan 313 may be provided inside the blowing part 310. Since the blowing part 310 is provided in the connection path F10, air flows in the connection path F10 when the blowing part 310 is driven, and since the connection path F10 also communicates with the accommodation space 101 of the inner cabinet 100, the air flows and moves in the accommodation space 101 by driving the blowing part 310.

In this way, the air may be sucked from the inner cabinet 100 into the connection path F10 by the driving of the blowing part 310 (a rotation of the blowing fan 313), and the air inside the connection path F10 may be ventilated.

In one embodiment, the blowing part 310 is provided inside the module housing 200 forming the connection path F10, and the blowing part 310 is driven to suction the air inside the inner cabinet 100 into an inlet 203. The air inside the connection path F10 passes through the module housing 200 constituting the connection path F10, the dry air duct 370, and a nozzle duct 810 and is then discharged back into the inner cabinet 820.

As such, the flow of air may be generated in the shoes care device 1 by the driving of the blowing part 310.

Dry air may be supplied to the inside of the inner cabinet 100 by the blowing part 310.

The heating part 320 is provided at one side of the dehumidifying part 330 and the blowing part 310 in the connection path F10. The heating part 320 may be provided inside the module housing 200. Based on a movement direction of the air inside the module housing 200, the module housing 200 may be arranged in an order of the blowing part 310, the heating part 320, and the dehumidifying part 330. That is, the air introduced into the outlet 203 of the module housing 200 moves along the connection path F10 by passing sequentially through the blowing part 310, the heating part 320, and the dehumidifying part 330.

The heating part 320 is disposed inside the module housing 200 and is configured to heat the air of the module chamber 210 inside the module housing 200.

The heating part 320 may be configured to heat the dehumidifying part 330. The heating part 320 may be configured to heat the dehumidifying material 331 constituting the dehumidifying part 330.

The air heated by the heating part 320 by the driving of the blowing part 310 moves directly to the dehumidifying part 330, thus heating the dehumidifying part 330. To this end, the heating part 320 is disposed inside the module housing 200 adjacent to the dehumidifying part 330. Specifically, the heating part 320 is disposed inside the module housing 200 adjacent to the dehumidifying part 330 based on a movement path of the air inside the module housing 200.

In the module housing 200, the dehumidification by the dehumidifying material 331 or the regeneration of the dehumidifying material 331 may be achieved by selectively heating the heating part 320.

The heating part 320 may be fixedly coupled to the module housing 200 in the module housing 200.

The heating part 320 may be made up of various devices and structures within a range capable of heating the air inside the module housing 200 or supplying heat to the dehumidifying part 330.

The heating part 320 may be formed of an electric heater 321. In one embodiment of the present invention, the heating part 320 may include the heater 321. The heater 321 includes a heating element, and may be configured to supply heat to the periphery while the heating element generates heat by supplied electric energy. The heater 321 may include a nichrome wire as the heating element.

The heater 321 of the heating part 320 may be formed in a ring shape, and the air may move by penetrating the center and surroundings of the ring-shaped heater 321 and be simultaneously heated. The heater 321 of the heating part 320 may be repeatedly formed in a second module chamber 213 along the movement direction of air.

The heater 321 of the heating part 320 may be formed in a circular ring shape or a rectangular ring shape.

The heating part 320 may include a heater flange 322 to which the heater 321 is fixed.

The heater flange 322 may be formed in the form of a metallic plate.

The heater flange 322 may be formed of a combination of flat plates in the second module chamber 213 along the movement direction of air. The heater flange 322 has a cross section that may be formed of a plate shape or a combination of plates in the second module chamber 213 along the movement direction of air (the second direction (Y direction)).

The heater flange 322 may include an outer flange 322a and an inner flange 322b.

The outer flange 322a may be formed in a tubular shape along the second direction (Y direction). An interior of the outer flange 322a is provided with a space to move air along the movement direction of air (a direction parallel to the second direction (Y direction)) in the second module chamber 213.

The inner flange 322b is fixed to the interior of the outer flange 322a. The inner flange 322b may include two or more plates crossing each other, and the heater 321 of the heating part 320 may be fixed to the inner flange 322b.

The heater flange 322 may be formed in various forms that fix the heater 321 of the heating part 320 and do not interfere with a flow of air moving through the second module chamber 213.

In one embodiment of the present invention, the blowing part 310, the heating part 320, the dehumidifying part 330, and the module housing 200 may form one set.

The set may be provided in a plural form. The shoes care device 1 according to one embodiment may be provided with two sets.

Such a set may be provided in each of the first management device 2a and the second management device 2b.

Such a set may form a drying module DM in the shoes care device 1 according to one embodiment of the present invention.

That is, in one embodiment of the present invention, the drying module DM may include the module housing 200, the blowing part 310, the heating part 320, and the dehumidifying part 330. Furthermore, the drying module DM is provided in each of the first management device 2a and the second management device 2b.

In the shoes care device 1 according to one embodiment of the present invention, a plurality of drying modules DM may be provided.

In the shoes care device 1 according to one embodiment of the present invention, a pair of drying modules DM may be provided. When the shoes care device 1 is provided with the pair of drying modules DM, one of the drying modules DM may form a 'drying module A (DM1) ' as a drying module of the first management device 2a, and the other may form a 'drying module B (DM2) ' as a drying module of the second management device 2b.

The 'drying module' described in one embodiment of the present invention may be understood to refer to each of the 'drying module A' and the 'drying module B' except as otherwise particularly limited.

In the shoes care device 1 according to one embodiment of the present invention, the drying module A (DM1) and the drying module B (DM2) may operate in different modes. When the drying module A DM1 operates in a moisture absorption mode, the drying module B DM2 may operate in a regeneration mode. Conversely, when the drying module A DM1 operates in the regeneration mode, the drying module B DM2 may operates in the moisture absorption mode.

The 'moisture absorption mode' described in the present invention means a case in which the dehumidifying part 330 adsorbs moisture in the air, and the 'regeneration mode' means a case in which the moisture adsorbed to the dehumidifying part 330 is separated by heating the dehumidifying part 330.

Naturally, both the drying module A DM1 and the drying module B DM2 may operate in the moisture absorption mode or may operate in the regeneration mode.

The module housing 200 may be fixedly coupled to a lower side of the inner cabinet 100. The module housing 200 may be detachably coupled to a lower side of the inner cabinet 100.

The module housing 200 includes the module chamber 210 that is a space having other components accommodated inside. That is, the module chamber 210 is a space inside the module housing 200 distinguished from an external space of the module housing 200. As described above, the module housing 200 forms part of the connection path F10, and accordingly, the module chamber 210 is configured to communicate with a space outside the module housing 200. The module chamber 210 communicates with the accommodation space 101 of the inner cabinet 100.

The module housing 200 may include a module case 201 and a module cover 202.

The module case 201 and the module cover 202 may be formed by injection molding, respectively, and may be assembled with each other after manufacturing to form the module housing 200.

The module case 201 is formed in the form of a container that is concave substantially downwards, and forms the module chamber 210 of the module housing 200.

The module case 201 may be configured in the form of a container opened upwards, and includes a module opening 201a.

In a plan view, an area of the module opening 201a may be larger than or equal to an area of the module chamber 210.

The module chamber 210 may include a first module chamber 212, a second module chamber 213, and a third module chamber 214. The module chamber 210 may include a suction module chamber 211.

In order to distinguish between the suction module chamber 211, the first module chamber 212, the second module chamber 213, and the third module chamber 214, a module partition wall 220 may be formed inside the module housing 200. Furthermore, the module partition wall 220 guides the movement of air so that air moves in a predetermined direction inside the module housing 200.

The suction module chamber 211 is a first space where air is introduced into the module housing 200.

The first module chamber 212 is a space in which the blowing part 310 is accommodated, the second module chamber 213 is a space in which the heating part 320 is accommodated, and the third module chamber 214 is a space in which the dehumidifying part 330 is accommodated.

In one embodiment of the present invention, the suction module chamber 211, the first module chamber 212, the second module chamber 213, and the third module chamber 214 may be formed at different positions in a plan view.

In addition, the air of the module chamber 210 may be configured to move the suction module chamber 211, the first module chamber 212, the second module chamber 213, and the third module chamber 214 sequentially. That is, when the blowing part 310 is driven, air moves sequentially through the suction module chamber 211, the first module chamber 212, the second module chamber 213, and the third module chamber 214 inside the module housing 200.

The module case 201 may include a dry air outlet 231 and a wet air outlet 232.

The dry air outlet 231 may be formed in a hole shape opened to allow air in the third module chamber 214 to flow out. The dry air outlet 231 is formed adjacent to the third module chamber 214. The dry air outlet 231 may be formed on one edge of the module case 201. Furthermore, the dry air outlet 231 may be connected to the accommodation space 101 through the dry air duct 370 and the nozzle duct 810.

The wet air outlet 232 may be formed in a hole shape opened to allow the air in the third module chamber 214 to flow out. The wet air outlet 232 is formed adjacent to the third module chamber 214. The wet air outlet 232 may be formed on a frame on one side of the module case 201. Furthermore, the wet air outlet 232 may be connected to the condenser 400.

The dry air outlet 231 and the wet air outlet 232 may be formed adjacent to each other. The dry air outlet 231 and the wet air outlet 232 may be formed adjacent to any one vertex part of the module housing 200.

The suction module chamber 211 is formed adjacent to the first module chamber 212, and a bottom surface of the suction module chamber 211 may be inclined downwardly toward the first module chamber 212. Accordingly, air introduced into the suction module chamber 211 may naturally move toward the first module chamber 212 by hitting the bottom surface of the suction module chamber 211 forming an inclined surface, and a condensed water introduced into the suction module chamber 211 may move along the bottom surface of the suction module chamber 211 forming the inclined surface, and may move to the first module chamber 212.

The blowing part 310 may be assembled to the module housing 200 while being spaced apart from a bottom surface of the first module chamber 212. Furthermore, in this case, the blowing part 310 may be configured such that air may be introduced from a lower side of the first module chamber 212 to an interior of the blowing part 310 inside the first module chamber 212.

The module case 201 may include a first condensed water discharge hole 233.

The first condensed water discharge hole 233 is formed in a hole shape penetrating the module case 201. The first condensed water discharge hole 233 is formed on an edge of the module case 201 adjacent to a condenser 400 and formed to be equal to or lower than the bottom surface of the first module chamber 212, and communicates with the condenser 400. Among the bottom surfaces of the first module chamber 212, the first condensed water discharge hole 233 may form the lowest part, or the bottom surface of the first module chamber 212 may be formed such that a height thereof is lowered toward or at least equal to the first condensed water discharge hole 233.

As such, the first condensed water discharge hole 233 may be lower than the bottom surface of the first module chamber 212, and accordingly, the condensed water introduced into the first condensed water discharge hole 232 may move toward the first condensed water discharge hole 233 and flow into the condenser 400 through the first condensed water discharge hole 233.

Meanwhile, since the first condensed water discharge hole 233 is a hole in which the module housing 200 and the condenser 400 communicate with each other, the air inside the condenser 400 may flow into the module housing 200 through the first condensed water discharge hole 233. The air introduced into the module housing 200 through the first condensed water discharge hole 233 from an interior of the condenser 400 may move along the first module chamber 212, the second module chamber 213, and the third module chamber 214 by an operation of the blowing part 310 and may be introduced again into the condenser 400 and condensed.

The shoes care device 1 according to one embodiment of the present invention includes the condenser 400 coupled to an outer surface of the inner cabinet 100 and forming a regeneration path F20. In a plan view, the first module chamber 212 may be provided between the second module chamber 213 and the condenser 400. Since the first module chamber 212 is disposed between the second module chamber 213 and the condenser 400, a direct heat exchange between the condenser 400 and the heating part 320 is blocked, and the heat may be prevented from being transferred to the condenser 400 when the heating part 320 is heated inside the second module chamber 213.

Accordingly, when the dehumidifying part 330 is regenerated, condensation depending on heating of air by the heater 321 of the heating part 320 and cooling of air inside the condenser 400 may be effective performed.

The dehumidifying part 330 may be coupled to the module housing 200 while being spaced apart from a bottom surface of the third module chamber 214. Furthermore, in this case, the air inside the third module chamber 214 may move downwards through the dehumidifying part 330 from an upper side of the third module chamber 214.

The module case 201 may include a second condensed water discharge hole 234.

The second condensed water discharge hole 234 is formed in a hole shape penetrating the module case 201. The second condensed water discharge hole 234 is formed on the edge of the module case 201 adjacent to the condenser 400 and formed to be equal to or lower than the bottom surface of the third module chamber 214, and communicates with the condenser 400. Among the bottom surfaces of the third module chamber 214, the second condensed water discharge hole 234 may form the lowest part, or the bottom surface of the third module chamber 214 may be formed such that a height thereof is lowered toward or at least equal to the second condensed water discharge hole 234.

The second condensed water discharge hole 234 may be formed adjacent to the wet air outlet 232.

In this way, the second condensed water discharge hole 234 may be lower than the bottom surface of the third module chamber 214, and accordingly, condensed water introduced into the third module chamber 214 may move toward the second condensed water discharge hole 234, and may flow into the condenser 400 through the second condensed water discharge hole 234.

Meanwhile, since the second condensed water discharge hole 234 is a hole in which the module housing 200 and the condenser 400 communicate with each other, the air inside the condenser 400 may flow into the module housing 200 through the second condensed water discharge hole 234. In this way, the air introduced from the interior of the condenser 400 into the module housing 200 through the second condensed water discharge hole 234 moves directly to the wet air outlet 232 by the driving of the blowing part 310, and may be introduced again into the condenser 400 and condensed.

The module housing 200 may include the module cover 202.

The module cover 202 is coupled to the module case 201 while shielding the module opening 201a from an upper side of the module case 201. The module cover 202 may be detachably coupled to the module case 201. A plurality of locking projections 292 may protrude from one of the module cover 202 and the module case 201, and a plurality of locking grooves 291 into which the locking projections 292 are inserted and locked may be formed on the other. The locking projections 292 and locking grooves 291 are provided in a plural form, respectively, and may be spaced apart along an edge of the module housing 200 and repeatedly formed.

With the blowing part 310, the heating part 320, and the dehumidifying part 330 accommodated in the module case 201, the module cover 202 may shield the blowing part 310, the heating part 320, and the dehumidifying part 330 and may be coupled to the module case 201.

The shoes care device according to one embodiment of the present invention may be formed in a structure in which the dehumidifying part 330 may be detachable from the module housing 200. The structure of the shoes care device provides an advantageous advantage in maintaining and managing the dehumidifying part 330 and the shoes care device 1 on the whole.

On the other hand, the dehumidifying part 330 may be repeatedly used by regeneration, but with the repeated use, the dehumidifying part 330 needs to be replaced.

Considering these descriptions, the shoes care device 1 according to one specific embodiment of the present invention may be configured to separate and replace the dehumidifying part 330.

In one embodiment of the present invention, the module cover 202 of the module housing 200 may form a bottom surface of the inner cabinet 100.

The module cover 202 may form a boundary surface between the inner cabinet 100 and the module housing 200. The module cover 202 may be formed in a substantially rectangular shape.

The module cover 202 may be configured in substantially parallel with the horizontal direction.

Alternatively, the module cover 202 may be inclined to any one side. In one embodiment, an upper surface of the module cover 202 may be inclined downwardly toward the first direction (X direction) (a front side of the shoes care device 1).

In one embodiment of the present invention, the main shelf 40 is mounted in close contact with an upper side surface of the module cover 202, and the main shelf 40 mounted on the upper side surface of the module cover 202 is also configured to be inclined when the upper side surface of the module cover 202 is inclined. In this case, since an upper surface of the main shelf 40 is inclined, water (e.g., condensed water) placed on the upper surface of the main shelf 40 may flow along an inclined direction.

The shoes care device 1 may include a dehumidifying material cover 241.

The dehumidifying material cover 241 forms part of the module cover 202 that is the bottom of the inner cabinet 100. Furthermore, the dehumidifying material cover 241 may be detached from the module cover 202 of the inner cabinet 100 or may be hinge-coupled to the module cover 202.

In the module cover 202, a dehumidifying material exit 240 which is an opening of a shape and a size corresponding to the dehumidifying material cover 241 may be formed. The dehumidifying material cover 241 may be configured to open and close the dehumidifying material exit 240. The dehumidifying material cover 241 may be tightly coupled to the dehumidifying material exit 240. At least a part of the dehumidifying material cover 241 may be separated from the module cover 202. In one embodiment, the dehumidifying material exit 240 of the module cover 202 may be opened while completely separating the dehumidifying material cover 241 from the module cover 202, and in another embodiment, the dehumidifying material cover 241 of the module cover 202 may be opened while rotating the dehumidifying material cover 241 around a hinge axis. The dehumidifying part 330 may be introduced into or withdrawn from the module housing 200 through the dehumidifying material exit 240.

The dehumidifying material exit 240 and the dehumidifying material cover 241 may be formed in a position corresponding to the third module chamber 214 in a plan view. That is, the dehumidifying material exit 240 and the dehumidifying material cover 241 may be formed directly above the third module chamber 214. The shoes care device 1 according to one embodiment of the present invention may be configured such that the first module chamber 212 and the second module chamber 213 are not exposed in a plan view in a state where the dehumidifier cover 241 is opened.

When the dehumidifying material cover 241 is opened in the module cover 202, the third module chamber 214 disposed on a lower part of the module cover 202 is exposed through the dehumidifying material exit 240 of the module cover 202, and the dehumidifying part 330 may be settled inside the module case 201, or may be immediately withdrawn and separated from the module case 201.

The sizes and shapes of the dehumidifying material cover 241 and the dehumidifying material exit 240 are variously provided within the range capable of withdrawing or inserting the dehumidifying part 330.

The dehumidifying material cover 241 may be formed in a rectangular plate shape.

The length of the dehumidifying material cover 241 in the first direction (X direction) may be equal to or longer than the length of the dehumidifying part 330, and the length of the dehumidifying material cover 241 in the second direction (Y direction) may be equal to or longer than the length of the dehumidifying part 330.

As described above, in the shoes care device 1 according to one embodiment of the present invention, the heating part 320 and the dehumidifying part 330 are formed at different positions in a top plane view, and when the dehumidifying material cover 241 is opened on the module cover 202 that forms the bottom surface of the inner cabinet 100, the dehumidifying part 330 disposed directly below the dehumidifying material cover 241 may be withdrawn from the module housing 200, and the dehumidifying part 330 may be easily replaced by the user.

In addition, since only the third module chamber 214 is exposed in a state in which the dehumidifier cover 241 is opened, and the first module chamber 212 and the second module chamber 213 are not exposed, the blowing part 310 accommodated in the first module chamber 212 and the heating part 320 accommodated in the second module chamber 213 are not exposed. That is, since the blowing part 310 and the heating part 320 are not directly exposed to the user, safety accidents due to an unintended operation of the blowing part 310 and/or the heating part 320 can be prevented.

The dehumidifying material cover 241 may be configured to separately shield the dehumidifying part 330. A space between the dehumidifying material cover 241 and the dehumidifying part 330 may form a part of the connection path F10.

As described above, the outlet 203 forms an inlet through which the air inside the inner cabinet 100 is sucked into the module housing 200. The outlet 203 may form a start part of the connection path F10. The outlet 203 may be formed in the shape of a hole vertically penetrated from the bottom surface (the upper surface of the module cover 202) of the inner cabinet 100.

A network such as a grid shape, a mesh shape, etc., may be formed on the outlet 203.

The outlet 203 may be formed parallel to the second direction (Y direction). That is, the outlet 203 may be formed in a long hole shape in the module cover 202 along the second direction (Y direction).

The outlet 203 may be formed on an edge of the module cover 202. The outlet 203 may be formed on the edge of the module cover 202 along the second direction (Y direction).

The outlet 203 may be formed on a front part or a rear part of the module cover 202 based on the first direction (X direction).

The outlet 203 may be disposed relatively close to the door 30 in the module cover 202. That is, the outlet 203 may be disposed relatively in the front of the module cover 202.

The upper surface of the module cover 202 may be inclined downwardly toward the outlet 203. That is, a part of the module cover 202 where the outlet 203 is formed may be configured to be the lowest. Accordingly, when water is present on the module cover 202 or the main shelf 40, such water may flow along the surface of the module cover 202 by gravity and flow into the inlet 203.

In the shoes care device 1 according to one embodiment of the present invention, the module chamber 210 is provided inside the module housing 200, and the module chamber 210 includes a first module chamber 212, a second module chamber 213, and the third module chamber 214. The first module chamber 212, the second module chamber 213, and the third module chamber 214 may be formed at different positions in a plan view. That is, the blowing part 310, the heating part 320, and the dehumidifying part 330 may be disposed at different positions in the module housing 200. According to one embodiment of the present invention, the blowing part 310, the heating part 320, and the dehumidifying part 330, which are main means for drying the air inside the inner cabinet 100 and main means for regenerating the dehumidifying part 330, are disposed together in the module chamber 210 of the module housing 200. Accordingly, the blowing part 310, the heating part 320, and the dehumidifying part 330 are disposed at positions considerably close to each other.

In one embodiment of the present invention, the module case 201 of the module housing 200 may be integrally formed by injection molding. In this case, the bottom parts of the module housing 200 may be integrally formed, the bottom parts may not be assembled with each other, and no gaps may be formed in the bottom parts.

In the arrangement explained above, the condensed water can be effectively prevented from leaking from the module housing 200. In addition, a vertical height of the module housing 200 can be minimized.

When moisture remains at an unintended part inside the shoes care device 1, such moisture may reproduce bacteria or cause odors. This is why there is need for countermeasures to solve the problems, and the shoes care device 1 according to one embodiment of the present invention can effectively prevent water from leaking in consideration of such problems.

In the shoes care device 1 according to one embodiment of the present invention, the air in the module chamber 210 may sequentially move the first module chamber 212, the second module chamber 213, and the third module chamber 214. Accordingly, since the third module chamber 214 and a drying flow path F10b may be connected in the shortest distance, which provides excellent drying efficiency by the dehumidifying part 330, and air heated by the heating part 320 moves directly to the dehumidifying part 330 to form the shoes care device 1 with excellent regeneration efficiency.

In the shoes care device 1 according to one embodiment of the present invention, the module housing 200 includes the suction module chamber 211, and the bottom surface of the suction module chamber 211 may be inclined downwardly toward the first module chamber 212. Accordingly, the air introduced through the outlet 203 moves naturally to the first module chamber 212 by hitting the bottom surface of the suction module chamber 211, and the condensed water introduced into the outlet 203 moves to the first module chamber 212 such that the condensed water can be easily drained.

The shoes care device 1 according to one embodiment of the present invention may include the condenser 400, and the module case 201 may include the first condensed water discharge hole 233. In addition, the module case 201 may include the second condensed water discharge hole 234. Accordingly, the dehumidifying part 330 may be effectively regenerated, and condensed water inside the module housing 200 may be easily discharged to the condenser 400.

In the shoes care device 1 according to one embodiment of the present invention, steam generated by the steam generator 700 is supplied to the accommodation space 101 of the inner cabinet 100, and to this end, the shoes care device 1 includes a steam inlet 204.

The steam inlet 204 forms an inlet through which steam is supplied to the accommodation space 101 of the inner cabinet 100.

In the shoes care device according to one embodiment of the present invention, the steam inlet 204 is formed in the module housing 200.

The steam inlet 204 may be formed in a rear part of the module housing 200 based on the first direction (X direction). The steam inlet 204 may be formed to vertically penetrate the module housing 200. The steam inlet 204 may be formed to vertically penetrate the module case 201 and the module cover 202. The steam inlet 204 may be formed in the center in a right-left direction at the rear part of the module housing 200.

The steam inlet 204 may be formed on a rear edge of the module housing 200, and may be formed directly behind a position where the third module chamber 214 is formed. The third module chamber 214 and the steam inlet 204 are shielded from each other.

In the module housing 200, the module cover 202 forms the bottom surface of the accommodation space 101, and when the module housing 200 is coupled to the inner cabinet 100, the steam inlet 204 is formed behind the bottom of the inner cabinet 100.

The steam generator 700 and the steam valve 710 are disposed in a lower part, the distance from the steam generator 710 to the steam inlet 204 can be reduced by forming the module housing 200, and the steam inlet 204 in a rear part of the module housing 200, and an increase in the load required for the supply of steam can be prevented. Accordingly, steam may be smoothly supplied from the steam generator 700 to the steam inlet 204

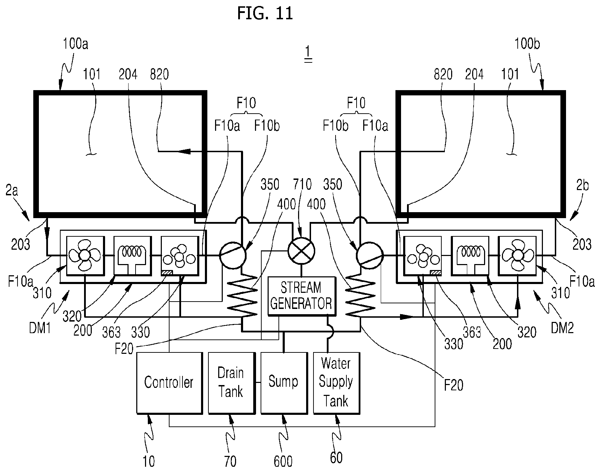

FIG. 11 is a diagram illustrating a connection relationship between components and a flow of fluid in the shoes care device 1 according to one embodiment of the present invention.

The connection path F10 forms a movement path of air connected from the outlet 203 to the nozzle 820. That is, the outlet 203 may form an inlet of the connection path F10, and the nozzle 820 may form an outlet of the connection path F10.

The outlet 203 may be coupled to communicate with the inner cabinet 100, and the nozzle 820 may be provided inside the inner cabinet 100. Except the outlet 203 and the nozzle 820, one part of the connection path F10 may be provided inside the inner cabinet 100, and the other part may be provided outside the inner cabinet 100.

The air inside the inner cabinet 100 moves to the connection path F10 through the outlet 203, and the air passing through the connection path F10 moves back into the inner cabinet 100 through the nozzle 820. As such air flow is repeated, the air circulation is performed in the shoes care device 1.

In the nozzle 820, a hole through which air is discharged is formed in the accommodation space 101 of the inner cabinet 100, and the nozzle 820 may form a last part of the connection path F10.

In the shoes care device 1 according to one embodiment of the present invention, since the nozzle 820 is configured to be movable to various positions inside the inner cabinet 100, the shoes may be managed in various positions.

As described above, the dehumidifying part 330 is disposed in the connection path F10. The air moving through the connection path F10 passes through the dehumidifying part 330, and the dehumidifying part 330 absorbs moisture from the air moving through the connection path F10 such that the air from which moisture has been removed may be supplied into the inner cabinet 100.

The connection path F10 may be divided into a conversion flow path F10a and a drying flow path F10b. The conversion flow path F10a and the drying flow path F10b form a movement path of air sequentially connected to each other. The air in the connection path F10 may sequentially move through the conversion flow path F10a and the drying flow path F10b.

The conversion flow path F10a forms an upstream section of the connection path F10, which is connected to the outlet 203. The conversion flow path F10a may be a section in which the blowing part 310, the heating part 320, and the dehumidifying part 330 are disposed. The conversion flow path F10a may be formed by the module housing 200, and the module chamber 210 inside the module housing 200 may form the conversion flow path F10a.

The conversion flow path F10a may be a section in which humid air moves and dries. The conversion flow path F10a may be a section in which air is dehumidified by the dehumidifying part 330.

Meanwhile, the conversion flow path F10a may be a section in which the dehumidifying part 330 (the dehumidifying material 331) are regenerated.

The drying flow path F10b forms a downstream section of the connection path F10, which connects the conversion flow path F10a to the nozzle 820. A flow path formed by the drying air duct 370, the nozzle duct 810, and the nozzle 820 may form the drying flow path F10b.

The drying passage F10b may be a section in which dry air with moisture removed therefrom moves.

When the drying module DM operates in the moisture absorption mode, the drying flow path F10b communicates with the conversion flow path F10a, and when the drying module DM operates in the regeneration mode, the drying flow path F10b and the conversion flow path F10a may not communicate with each other such that the drying flow path F10b and the conversion flow path F10a block each other.

Accordingly, when air is dehumidified by the dehumidifying part 330 in the conversion flow path F10a, the dried air moves through the drying flow path F10b.

The dry air duct 370 may be fixedly coupled to an outer wall surface of the inner cabinet 100, and the nozzle duct 810 may be provided inside the inner cabinet 100.

As the dry air duct 370 is tightly coupled to an inner rear plate 110 of the inner cabinet 100, a flow path may be formed between the dry air duct 370 and the inner cabinet 100 (the inner rear plate 110), and such a flow path may form a part of the drying flow path F10b. A lower part of the dry air duct 370 communicates with the dry air outlet 231 of the module housing 200, an upper part thereof communicates with the nozzle duct 810, which connect the interior of the module housing 200 and the interior of the nozzle duct 810 for mutual communication.

As described above, after humid air in the accommodation space 101 of the inner cabinet 100 flows into the conversion flow path F10a, the air is dehumidified by the dehumidifying part 330 and converted into dry air, and the dry air can be resupplied to the accommodation space 101 of the inner cabinet 100 through the drying flow path F10b.

The regeneration path F20 forms a movement path of a fluid.

The regeneration path F20 forms a passage through which air and/or condensed water inside the shoes care device moves.

The regeneration path F20 forms a path through which air and/or condensed water passing through the dehumidifying part 330 moves when the dehumidifying material 331 is regenerated. The regeneration path F20 may be entirely or partially formed of a pipe, a hose, a tube, a duct, a housing, or a combination thereof.

Moisture generated during the regeneration process of the dehumidifying material 331 needs to be discharged through a separate flow path separated from the drying flow path F10b, which is a flow path through which dry air moves. Accordingly, the shoes care device 1 according to one embodiment of the present invention includes the regeneration path F20, and when the dehumidifying material 331 is regenerated, air passing through the dehumidifying part 330 is not ventilated to the nozzle 820 but moves through the regeneration path F20.

The regeneration path F20 is a flow path branched from the connection path F10. The regeneration path F20 may be branched from the connection path F10 to form a path different from that of the drying flow path F10b of the connection path F10. The regeneration path F20 is connected to the sump 600.

The regeneration path F20 may be a section that connects the conversion flow path F10a and the sump 600.

The regeneration path F20 may be a section in which humid air separated from the dehumidifying part 330 moves.

The condenser 400 according to one embodiment of the present invention forms a regeneration path F20. Moisture separated from the dehumidifying material 331 may be condensed after moving to the condenser 400 along with air moving along the regeneration path F20. In addition, condensed water condensed in the condenser 400 may be moved to the sump 600 through the regeneration path F20, collected from a lower part of the sump 600, and then discharged to the drain tank 70, discharged to the outside, or pressed to the steam generator 700.

In the shoes care device 1 according to one embodiment of the present invention, when the drying module DM operates in the regeneration mode, the regeneration path F20 communicates with the conversion flow path F10a, and when the drying module DM operates in the moisture absorption mode, the regeneration path F20 and the conversion flow path F10 may not communicate with each other such that the regeneration path F20 and the conversion flow path F10 block each other.

Accordingly, when the dehumidifying part 330 is regenerated in the conversion flow path F10a, humid air containing moisture separated from the dehumidifying part 330 moves through the regeneration path F20.

In one embodiment of the present invention, the damper 350 may be formed in the form of a damper valve.

The damper 350 may be rotatably coupled to the module housing 200. The damper 350 may be coupled to the module housing 200 in a form accommodated in the module housing 200.

As described above, in the module housing 200, the dry air outlet 231 forming an inlet of the drying flow path F10b is formed as a passage of the connection path F10, and the wet air outlet 232 forming an inlet of the regeneration path F20 is formed.

The damper 350 controls a movement path of air passing through the dehumidifying material 331 in the module housing 200. Depending on the operation of the damper 350, the air passing through the dehumidifying material 331 may move into the inner cabinet 100 through the nozzle 820 or may move into the regeneration path F20.

The damper 350 may be configured to open the regeneration path F20 while blocking the drying flow path F10b, or to open the drying flow path F10b while blocking the regeneration path F20.

The damper 350 may be configured to selectively shield the dry air outlet 231 and the wet air outlet 232. The damper 350 may be configured to selectively seal the dry air outlet 231 and the wet air outlet 232.

The damper 350 may selectively block one of the dry air outlet 231 and the wet air outlet 232. When the damper 350 opens the dry air outlet 231 while blocking the wet air outlet 232, the air passing through the dehumidifying material 331 may move into the inner cabinet 100 through the nozzle 820, and when the damper 350 opens the wet air outlet 232 while blocking the dry air outlet 231, the air passing through the dehumidifying material 331 may be condensed while moving through the regeneration path F20.

In the shoes care device 1 according to one embodiment of the present invention, the damper 350 may be configured to be hinge-rotatable around a hinge axis 350a formed on one side. The hinge axis 350a of the damper 350 may be parallel to the third direction (Z direction). In addition, the shoes care device 1 may include a damper motor 351 configured to rotate the damper 350 around the hinge axis 350a of the damper 350. The damper motor 351 may be formed as an electric motor and may be configured to rotate the damper 350 bidirectionally.

When the damper 350 opens the dry air outlet 231 and seals the wet air outlet 232, the air inside the inner cabinet 100 moves along the connection path F10 and is circulated by sequentially passing through the inlet 203, the module housing 200 (the blowing part 310 and the dehumidifying part 330, the dry air outlet 231, the dry air duct 370, the nozzle duct 810, and the nozzle 820.

When the damper 350 seals the dry air outlet 231 and opens the wet air outlet 232, the air moves along the conversion flow path F10a and the regeneration path F20 and is circulated by sequentially passing through the module housing 200 (the blowing part 310, the heating part 310, and the dehumidifying part 330), the wet air outlet 232, and the condenser 400.

In one embodiment of the present invention, the controller 10 may control the damper motor 351 such that the damper 350 closes the dry air outlet 231 and opens the wet air outlet 232 when the heating part 320 is turned on. In addition, the controller 10 may control the damper motor 351 such that the damper 350 opens the dry air outlet 231 and closes the wet air outlet 232 when the heating part 320 is turned off.

Accordingly, by controlling the damper motor 351 by the controller 10, the damper 350 may open the drying flow path F10b and close the regeneration path F20 when the heating part 320 is turned off, and may close the drying flow path F10b and open the regeneration path F20 when the heating part 320 is turned on.

The damper motor 351 may be controlled individually in each of the first management device 2a and the second management device 2b.

Referring to FIG. 11, in the drying module A (DM1) of the first management device 2a, the damper 350 seals the wet air outlet 232 and opens the dry air outlet 231 of the second management device 2b, and in the drying module B (DM2) of the second management device 2b, when the damper 350 opens the wet air outlet 232 and seals the dry air outlet 231, the air in the conversion flow path F10a of the first management device 2a may flow through the drying flow path F10b, and the air in the conversion flow path F10a of the second management device 2b may flow through the regeneration path F20. Furthermore, in this case, the drying module A (DM1) of the first management device 2a may operate in the moisture absorption mode, and the drying module B (DM2) of the second management device 2b may operate in the regeneration mode.

In contrast, when in the drying module A (DM1) of the first management device 2a, the damper 350 opens the wet air outlet 232 and seals the dry air outlet 231, and in the drying module B (DM2) of the second management device 2b, the damper 350 seals the wet air outlet 232 and opens the dry air outlet 231, the air in the conversion flow path F10a of the first management device 2a may flow along the regeneration path F20, and the air in the conversion flow path F10a of the second management device 2b may flow along the drying flow path F10b. Furthermore, in this case, the drying module A (DM1) of the first management device 2a may operate in the regeneration mode, and the drying module B (DM2) of the second management device 2b may operate in the moisture absorption mode.

In both the drying module A (DM1) of the first management device 2a and the drying module B (DM2) of the second management device 2b, when the damper 350 seals the wet air outlet 232 and opens the dry air outlet 231, both the drying module A DM1 and the drying module B (DM2) may operate in the moisture absorption mode.

In both the drying module A (DM1) of the first management device 2a and the drying module B (DM2) of the second management device 2b, when the damper 350 seals the dry air outlet 231 and opens the wet air outlet 232, both the drying module A (DM1) and the drying module B (DM2) may operate in the regeneration mode.

In the shoes care device 1 according to one embodiment of the present invention, the first management device 2a and the second management device 2b individually include the inner cabinet 100, the connection path F10, the blowing part 310, and the dehumidifying part 330. In addition, the shoes care device 1 includes the steam generator 700 and the steam valve 710. Accordingly, the degree of the supply of steam, the degree of dehumidification by the dehumidifying part 330, and the flow of air circulated along the connection path F10 may be different in each of the first management device 2a and the second management device 2b, and the first management device 2a and the second management device 2b may manage shoes under different conditions.

In addition, the first management device 2a and the second management device 2b include the module housing 200, the blowing part 310, the heating part 320, the dehumidifying part 330, and the drying flow path F10b, respectively. The air and condensed water moving in the first management device 2a and the air and condensed water moving in the second management device 2b move along different paths, thereby achieving accurate control intended in each of the first management device 2a and the second management device 2b. In this case, since the first management device 2a and the second management device 2b share and use the steam generator 700, the steam generator 700 can be efficiently utilized in the shoes care device 1, and the shoes care device 1 can efficiently utilize the space.

In addition, as described above, when the shoes are dried in one of the first management device 2a and the second management device 2b, the dehumidifying part 330 may be regenerated in the other, and the efficient management of the shoes and efficient use of the shoes care device 1 can be achieved.

In the shoes care device 1 according to one embodiment of the present invention, the first management device 2a and the second management device 2b each include the regeneration path F20 and the damper 350 individually.

The controller 10 may control the heating part 320 (the heater 321) and the damper 350 to interwork with each other.

The controller 10 may control the damper 350 to open the drying flow path F10b and close the regeneration path F20 when the heating part 320 is turned off, and may control the damper 350 to close the drying flow path F10b and open the regeneration path F20 when the heating part 320 is turned on.

The controller 10 may control each component of the shoes care device 1 such that air flowing into the module chamber 210 from the accommodation space 101 and passing through the dehumidifying part 330 moves along the drying flow path F10b when the heating part 320 is turned off (when the heater 321 of the heating part 320 is turned off), and the air moves along the regeneration path F20 when the heating part 320 is turned on (when the heater 321 of the heating part 320 is turned on).

Such control may be performed individually in each of the first management device 2a and the second management device 2b. Accordingly, since the movement path of air inside the module chamber 210 is changed depending on an operation of the heating part 320, the drying of the shoes and the regeneration of the dehumidifying part 330 can be effectively achieved.

The controller 10 may control the steam valve 710 and the heating part 320 to interwork with each other.

The controller 10 ma control the steam valve 710 such that when the heating part 320 of the first management device 2a is turned off, the valve disk 715 closes or opens the first valve outlet 713, when the heating part 320 of the first management device 2a is turned on, the valve disk 715 closes the first valve outlet 713, when the heating part 320 of the second management device 2b is turned off, the valve disk 715 closes or opens the second valve outlet 714, and when the heating part 320 of the second management device 2b is turned on, the valve disk 715 closes the second valve outlet 714.

In this way, the supply of steam to the accommodation space 101 of the inner cabinet 100 and the operation of the heating part 320 inside the module housing 200 may interwork with each other to effectively perform the drying of the shoes and the regeneration of the dehumidifying part 330.

The shoes care device 1 according to one embodiment of the present invention may include a first sensor 361 and a second sensor 362 (see FIG. 9).

The first sensor 361 may be installed in the second module chamber 213 of the module housing 200, and the second sensor 362 may be installed in the third module chamber 214 of the module housing 200. The first sensor 361 may be configured to measure the temperature and/or humidity of the second module chamber 213, and the second sensor 362 may measure the temperature and/or humidity of the third module chamber 214.

The first sensor 361 measures the temperature and/or humidity of air before passing through the dehumidifying part 330, and the second sensor 362 measures the temperature and/or humidity of air after passing through the dehumidifying part 330.

The controller 10 may compare the temperature and/or humidity of the second module chamber 213 measured by the first sensor 361 with the temperature and/or humidity of the third module chamber 214 measured by the second sensor 362 to recognize a state and a change of the temperature and/or humidity inside the module housing, and may also check an operation state of the drying module DM.

The controller 10 may recognize the temperature and humidity of the second module chamber 213 measured by the first sensor 361, and the temperature and humidity of the third module chamber 214 measured by the second sensor 362 to recognize a change in the humidity inside the module housing 200. Accordingly, the degree of dehumidification by the dehumidifying part 330 may be checked, and the degree of regeneration of the dehumidifying part 330 may be checked.

In one embodiment, when the drying module DM operates in the moisture absorption mode, the controller 10 may control the drying module DM to stop operating in the moisture absorption mode and operate in the regeneration mode if a humidity change amount of the second module chamber 213 recognized by the first sensor 361 and a humidity change amount of the third module chamber 214 recognized by the second sensor 362 is less than or equal to a reference value.

In one embodiment, when the drying module DM operates in the regeneration mode, the controller 10 may control the drying module DM to stop operating in the regeneration mode if the humidity change amount of the second module chamber 213 recognized by the first sensor 361 and the humidity change amount of the third module chamber 214 recognized by the second sensor 362 is less than or equal to the reference value.

The shoes care device 1 according to one embodiment of the present invention further includes a third sensor 363 capable of measuring the amount of moisture adsorbed to the dehumidifying material 331, and the controller 10 may control the drying module DM to operate the regeneration mode until the amount of moisture measured by the third sensor 363 is less than or equal to a set value.

Specifically, the controller 10 may control all the heating parts 320 to operate until the amount of moisture measured by the third sensor 363 is less than or equal to the set value.

In this case, as illustrated in FIG. 11, the third sensor 363 may include a moisture sensor installed adjacent to the dehumidifying material 331 to measure the amount of moisture adsorbed to the dehumidifying material 331, and the type and number thereof may vary as necessary.

In this way, when the moisture adsorbed on the dehumidifying material 331 is sensed to exceed the reference value, the shoes care device 1 according to one embodiment of the present invention first regenerates all dehumidifying materials 331 until the moisture is less than or equal to the reference value. Accordingly, the dehumidifying material 331 can maintain an appropriate state for dehumidification all the times even when the shoes care device 1 operates to refresh the shoes.

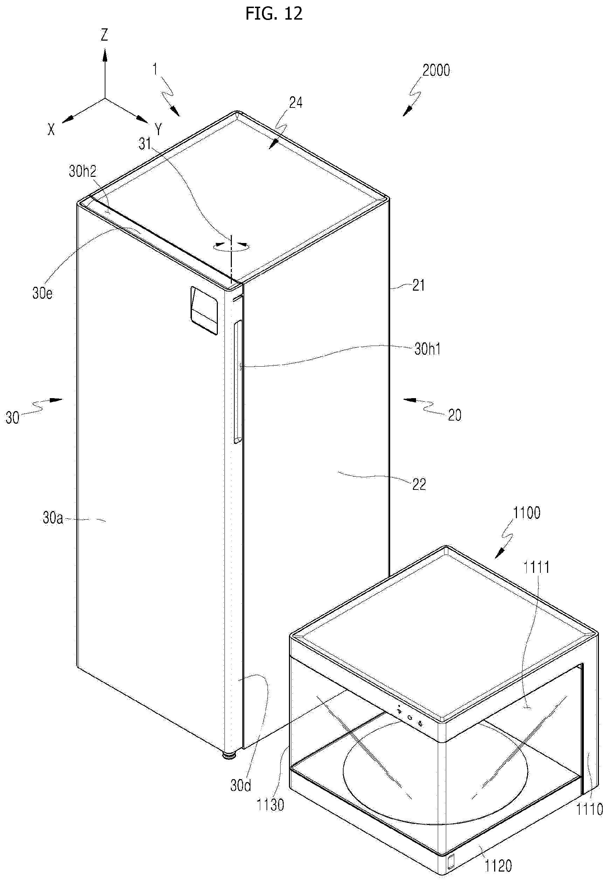

FIG. 12 is a perspective view illustrating the shoes care device 1 and a visible shoes care device 1100 of a shoes management set 1000 according to one embodiment of the present invention.

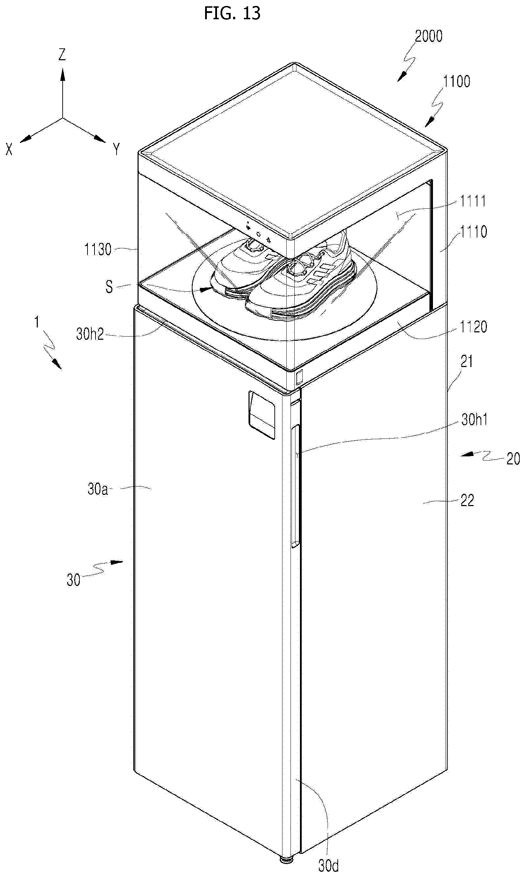

FIG. 13 is a perspective view illustrating a state in which the visible shoes care device 1100 of the shoes management set 1000 of FIG. 12 is stacked on an upper surface of the shoes care device 1.

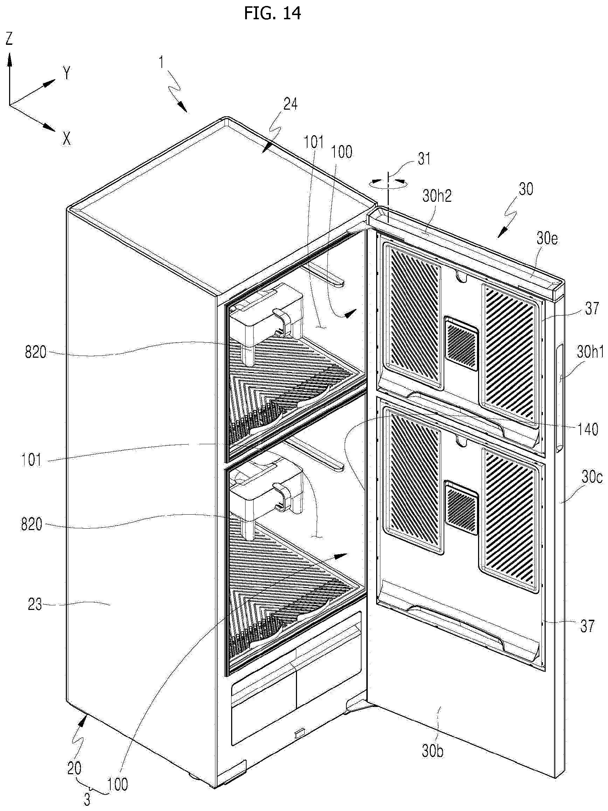

FIG. 14 is a perspective view of the shoes care device 1 of FIG. 12 when viewed from another direction, illustrating a state in which the door 30 is opened.

The shoe management set 1000 according to one embodiment of the present invention may include the shoes care device 1 and the visible shoes care device 1100.

The shoes care device 1 may form the accommodation space 101 in which air is forcibly circulated to treat the shoes. The shoes care device 1 may include a cabinet 3 and the door 30.

The cabinet 3 may include the inner cabinet 100 and the outer cabinet 20.

The inner cabinet 100 may form the accommodation space 101 inside. The inner cabinet 100 may have the main opening 140 that opens the accommodation space 101 in the first direction (X direction) as the horizontal direction. In one embodiment, the inner cabinet 100 may form two accommodation spaces 101. The two accommodation spaces 101 may be vertically spaced apart from each other.

In one embodiment, the first direction (X direction) may be a direction from the rear of the shoes care device 1 to the front. The first direction (X direction) may be parallel to the front-rear direction.

In addition, hereinafter, except for a particularly limited case, it will be described that the second direction (Y direction) is parallel to the left-right direction, and the third direction (Z direction) is parallel to the vertical direction.

The outer cabinet 20 may include the outer rear plate 21, the first outer side plate 22, the second outer side plate 23, and an outer upper plate 24.

The outer rear plate 21, the first outer side plate 22, the second outer side plate 23, and the outer upper plate 24 may be formed integrally with each other. Alternatively, the outer rear plate 21, the first outer side plate 22, the second outer side plate 23, and the outer upper plate 24 may be individually formed.

The outer upper plate 24 may form an upper surface of the cabinet 3. At least a part of the outer upper plate 24 may form a surface orthogonal to the third direction (Z direction). The outer upper plate 24 may form an upper wall surface of the outer cabinet 20 in the third direction (Z direction).

The door 30 may be configured to open and close the main opening 140. In shoes care device 1, the door 30 may be hinge-coupled to the cabinet 3. The hinge rotation axis 31 of the door 30 may be formed in the vertical direction. That is, in shoes care device 1, the door 30 may be configured to be rotatable bidirectionally around the rotation axis 31 in the vertical direction.

The door 30 may include the front plate 30a, the rear plate 30b, a first side plate 30c, a second side plate 30d, and an upper plate 30e. The front plate 30a may form the front surface of the shoes care device 1 in a state where the door 30 closes the accommodation space 101. The rear plate 30b may form a surface for closing the accommodation space 101 in a state where the door 30 closes the accommodation space 101.

The packing 37 may be coupled to the rear plate 30b. With the door 30 closing the accommodation space 101, the packing 37 may be compressed between the rear plate 30b and the front surface of the inner cabinet 100 along the circumference of the main opening 140. Accordingly, with the door 30 closing the accommodation space 101, an upper surface of the door 30 and the upper surface of the cabinet 3 may be spaced apart by a predetermined distance by the packing 37.

A concave groove (hereinafter, referred to as a 'first concave groove 30h1') may be formed in the first side plate 30c and the second side plate 30d. The user may pull and open the door 30 by applying force to the inner surface of the first concave groove 30h1. A concave groove (hereinafter, referred to as a 'second concave groove 30h2') may be formed in the upper plate 30e. The user may pull and open the door 30 by applying force to the inner surface of the second concave groove 30h2.

The outer cabinet 20 and the door 30 may form an overall appearance of the shoes care device 1. The outside of the shoes care device 1 may be formed in a hexahedral shape. That is, in a state where the outer cabinet 20 and the door 30 are coupled to each other and the door 30 is closed, the appearance of the shoes care device 1 may be formed in a hexahedral shape.

The shoes care device 1 may include the nozzle 820. The nozzle 820 may be provided in the accommodation space 101. The nozzle 820 may inject the forcibly ventilated air while being inserted into the shoes.

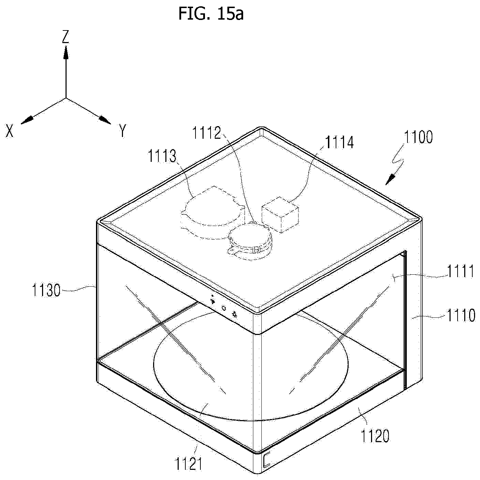

FIG. 15a is a perspective view illustrating the visible shoes care device 1100 of FIG. 12.

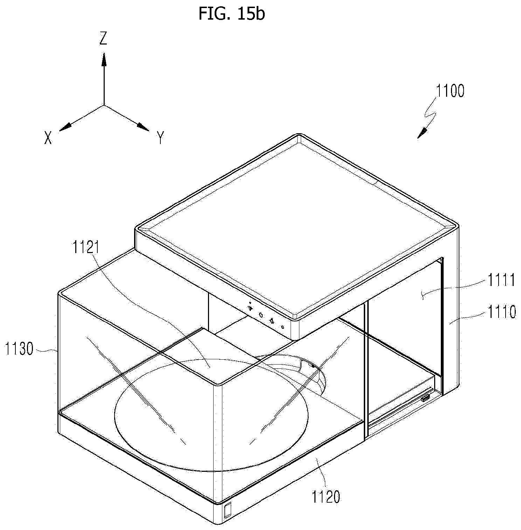

FIG. 15b is a view illustrating a state in which a display space 1111 of the visible shoes care device 1100 of FIG. 15 is opened.

The visible shoes care device 1100 may have the display space 1111 formed inside such that the shoes are displayed. The visible shoes care device 1100 may include a main body 1110, a moving body 1120, and a transparent window 1130.

The main body 1110 may form the display space 1111. In one embodiment, the main body 1110 may form a substantially '⊏' shape. The display space 1111 may form a substantially hexahedral shape.

The main body 1110 may form boundaries of three of the six surfaces of the display space 1111. That is, the main body 1110 may form boundaries of a rear surface in the first direction (X direction), and an upper surface and a lower surface in the third direction (Z direction), among the six surfaces of the display space 1111.

An upper part of the main body 1110 may be provided with a light 1112, a fan 1113 and a heater 1114.

The light 1112 may illuminate the display space 1111. The air forcibly ventilated by the fan 1113 may be introduced into the display space 1111 through a ventilation outlet 1115. Air in the display space 1111 may be introduced into the fan 1113 through a vent 1116. Accordingly, the air forcibly ventilated by the fan 1113 may be circulated in the display space 1111.

The heater 1114 may transfer thermal energy to the air forcibly ventilated by the fan 1113. The temperature of the display space 1111 may be adjusted by the heater 1114.

The moving body 1120 may include a turntable 1121. The shoes may be settled on the turntable 1121. A lower part of the moving body 1120 may form a shape surrounding the lower part of the main body 1110. The moving body 1120 may form a substantially '⊏' shape.

The moving body 1120 may be slidably coupled to the main body 1110 in a direction parallel to the first direction (X direction). For example, a rail parallel to the first direction (X direction) may be formed in the main body 1110. Furthermore, the moving body 1120 may be provided with a slider that slides along the rail.

The moving body 1120 may include the transparent window 1130. The transparent window 1130 may form a surface in contact with the display space 1111. The transparent window 1130 may form a substantially '⊏' shape.

The transparent window 1130 may form boundaries of three of the six surfaces of the display space 1111. That is, the transparent window 1130 may form boundaries the front surface in the first direction (X direction) and opposites surfaces in the second direction (Y direction), among the six surfaces of the display space 1111.

As illustrated in FIG. 15A, the moving body 1120 may move backward in the first direction (X direction) to close the display space 1111. As illustrated in FIG. 15b, the moving body 1120 may slide in the first direction (X direction) to open the display space 1111.

The user may insert the shoes into the display space 1111 through a gap between the main body 1110 and the moving body 1120. The user may withdraw the shoes accommodated in the display space 1111 through the gap between the main body 1110 and the moving body 1120.

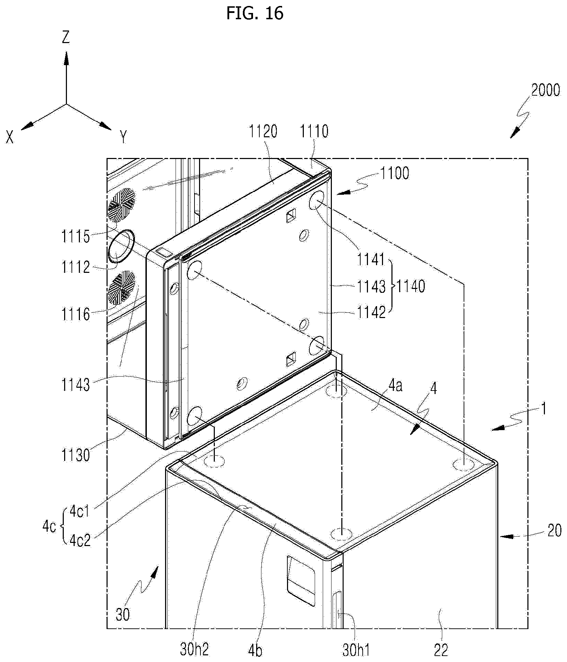

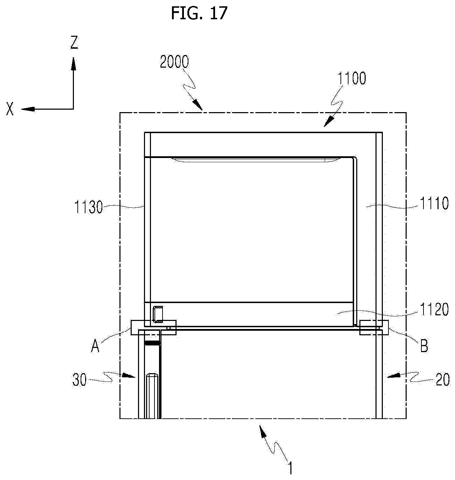

FIG. 16 is a perspective view illustrating a bottom surface of the visible shoes care device 1100 and the upper surface of the shoes care device 1, which constitutes the shoes management set 1000 of FIG. 12. FIG. 17 is a partial side view of the shoes management set 1000 of FIG. 13.

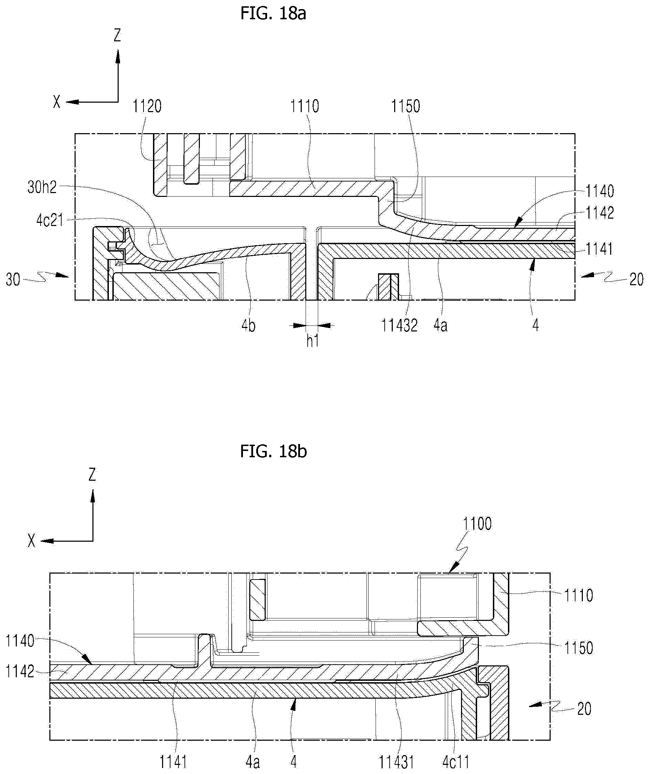

FIG. 18a is a partial cross-sectional view of the shoes management set 1000 of FIG. 17 cut in an XZ plane and is a view illustrating Part A of FIG. 17. FIG. 18b is a partial cross-sectional view of the shoes management set 1000 of FIG. 17 cut into the XZ plane and is a view illustrating Part B of FIG. 17.

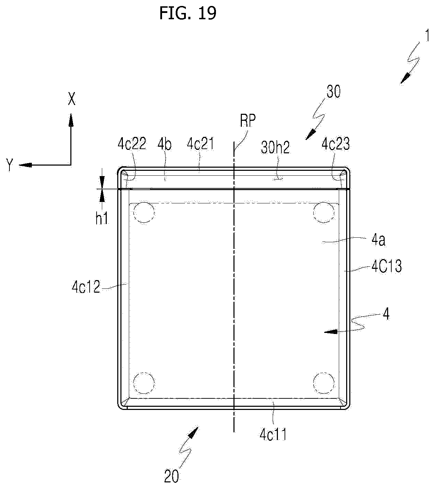

FIG. 19 is a top plan view illustrating the upper surface of the shoes care device 1 of FIG. 12.

The accommodation portion 4 recessed downwards may be formed on the upper surface of the shoes care device 1. The accommodation portion 4 may form a symmetrical shape with respect to the reference plane RP parallel to the first direction (X direction) and the vertical direction.

The cabinet 3 and the door 30 may form the accommodation portion 4 together. The outer upper plate 24 may form the upper surface of the cabinet 3. The upper plate 30e may form the upper surface of the door 30. Accordingly, the outer upper plate 24 and the upper plate 30e may form the accommodation portion 4 together.

The accommodation portion 4 may include an accommodation bottom portion 4a, an accommodation extension portion 4b, and an accommodation side portion 4c.

The accommodation bottom portion 4a may be formed on the upper surface of the cabinet 3. The accommodation bottom portion 4a may form a surface on which an article may be settled. In one embodiment, the accommodation bottom portion 4a may form a plane parallel to a horizontal plane. In one embodiment, the accommodation bottom portion 4a may have a substantially rectangular shape.

Here, the article may mean an object having a constant volume. For example, the article may refer to an electronic product, household goods, office goods, exhibition goods, etc. In one embodiment, as the accommodation bottom portion 4a forms a plane parallel to the horizontal plane, various article can be stably maintained with the articles settled in the accommodation bottom part 4a. In one embodiment, the article may refer to the visible shoes care device 1100.

The accommodation extension portion 4b may be formed on the upper surface of the door 30. With the door 30 closing the accommodation space 101, the upper surface of the door 30 and the upper surface of the cabinet 3 may be spaced apart from each other by a predetermined distance h1 by the packing 37. Accordingly, the accommodation extension portion 4b and the accommodation bottom portion 4a may be spaced apart from each other by the distance of h1 in a direction parallel to the first direction (X direction).

The accommodation extension portion 4b may form a surface adjacent to the accommodation bottom portion 4a, which has the same height as the accommodation bottom portion 4a. Accordingly, even if the visible shoes care device 1100 (or the article) settled in the accommodation bottom portion 4a is slightly pushed to the accommodation extension portion 4b due to external force or tilting of the shoes case device 1, the visible shoes care device 1100 (or the article) can be prevented from being reversed due to the height difference between the accommodation bottom portion 4a and the accommodation extension portion 4b.

The accommodation side portion 4c may form an edge of the accommodation portion 4. The accommodation side portion 4c may form a height greater than that of the accommodation bottom portion 4a. The accommodation side portion 4c may include a first accommodation side portion 4c1 and a second accommodation side portion 4c2.

The first accommodation side portion 4c1 may be formed on the upper surface of the cabinet 3 along the edge of the accommodation portion 4. The first accommodation side portion 4c1 may form a surface connected to the accommodation bottom portion 4a. The first accommodation side portion 4c1 may include an eleventh accommodation side portion 4c11, a twelfth accommodation side portion 4c12, and a thirteenth accommodation side portion 4c13.

The eleventh accommodation side portion 4c11 may be formed in the rear part in the first direction (X direction) with respect to the accommodation bottom portion 4a. The eleventh accommodation side portion 4c11 may be formed in the second direction (Y direction). As the eleventh accommodation side portion 4c11 goes to the edge of the accommodation portion 4, that is, moves away from the accommodation bottom portion 4a to the rear part in the first direction (X direction), it may rise upwards in the third direction (Z direction).

In the process in which the user settles the visible shoes care device 1100 (or the article) on the accommodation bottom portion 4a, the eleventh accommodation side portion 4c11 may form an inclined surface guiding the bottom surface of the visible shoes care device 1100 (or the article) to the accommodation bottom portion 4a, that is, the first direction (X direction).

In addition, when the visible shoes care device 1100 (or the article) settled in the accommodation bottom portion 4a is pushed to the eleventh accommodation side portion 4c11 due to the external force or the tilting of the shoes case device 1, the eleventh accommodation side portion 4c11 may form a boundary where the visible shoes care device 1100 (or article) is pushed to the rear part in the first direction (X direction) due to the height difference from the accommodation bottom portion 4a.

Accordingly, various visible shoes care devices 1100 (or articles) settled in the accommodation bottom portion 4a can stably maintain a state of being settled in the accommodation bottom portion 4a.

In addition, as the eleventh accommodation side portion 4c11 moves to the edge of the accommodation portion 4, that is, gets away from the accommodation bottom portion 4a to the rear in the first direction (X direction), the gradient may increase.

Accordingly, even if the visible shoes care device 1100 (or article) settled in the accommodation bottom portion 4a is slightly pushed to the eleventh accommodation side portion 4c11 due to the external force or the tilting of the shoes case device 1, the visible shoes care device 1100 (or the article) can be prevented from being reversed due to the height difference between the accommodation bottom portion 4a and the eleventh accommodation side portion 4c11.

In addition, the eleventh accommodation side portion 4c11 can stably form a boundary where the visible shoes care device 1100 (or the article) is pushed to the rear part in the first direction (X direction).

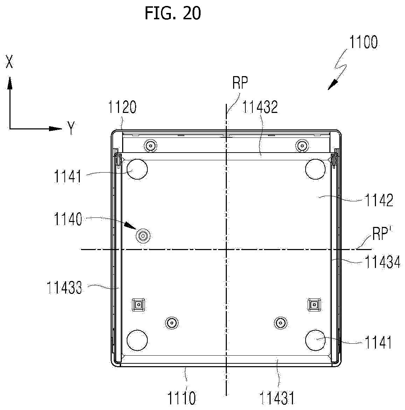

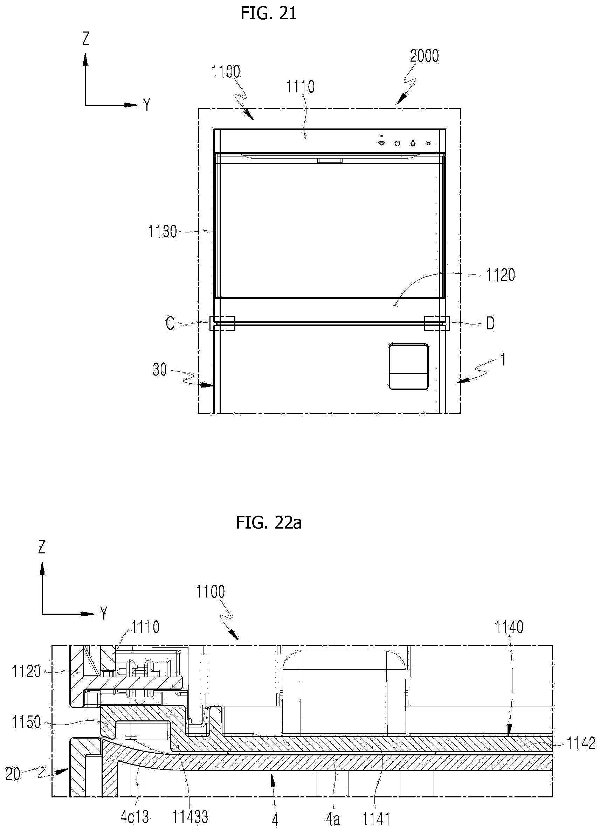

FIG. 20 is a bottom view illustrating a bottom surface of the visible shoes care device 1100 of FIG. 12. FIG. 21 is a partial front view of the shoes management set 1000 of FIG. 13.

FIG. 22a is a partial cross-sectional view of the shoes management set 1000 of FIG. 21 cut into an YZ plane and is a view illustrating Part C of FIG. 21

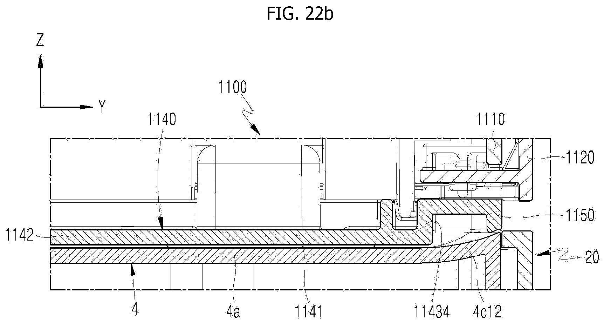

FIG. 22b is a partial cross-sectional view of the shoes management set 1000 of FIG. 21 cut into the YZ plane and is a view illustrating Part D of FIG. 21.

The twelfth accommodation side portion 4c12 may be formed on one side in the second direction (Y direction) with respect to the accommodation bottom portion 4a. The twelfth accommodation side portion 4c12 may be formed in the first direction (X direction). As the twelfth accommodation side portion 4c12 goes to the edge of the accommodation portion 4, that is, moves away from the accommodation bottom portion 4a to one side in the second direction (Y direction), it may rise upwards in the third direction (Z direction).

In the process in which the user settles the visible shoes care device 1100 (or the article) on the accommodation bottom portion 4a, the twelfth accommodation side portion 4c12 may form an inclined surface guiding the bottom surface of the visible shoes care device 1100 (or the article) to the accommodation bottom potion 4a, that is, to the other side in the second direction (Y direction).

In addition, when the visible shoes care device 1100 (or the article) settled in the accommodation bottom portion 4a is pushed to the twelfth accommodation side portion 4c12 due to the external force or the tilting of the shoes case device 1, the twelfth accommodation side portion 4c12 may form a boundary where the visible shoes care device 1100 (or the article) is pushed to the rear part in the first direction (X direction) due to the height difference from the accommodation bottom portion 4a.

Accordingly, various visible shoes care devices 1100 (or articles) settled in the accommodation bottom portion 4a can stably maintain the state of being settled in the accommodation bottom portion 4a.

In addition, the twelfth accommodation side portion 4c12 may form a surface continuous with the surface of the accommodation bottom portion 4a. Further, as the twelfth accommodation side portion 4c12 moves to the edge of the accommodation portion 4, that is, gets away from the accommodation bottom portion 4a to one side in the second direction (Y direction), the gradient may increase.

Accordingly, even if the visible shoes care device 1100 (or the article) settled in the accommodation bottom portion 4a is slightly pushed to the twelfth accommodation side portion 4c12 due to the external force or the tilting of the shoes case device 1, the visible shoes care device 1100 (or the article) may be prevented from being reversed due to the height difference between the accommodation bottom portion 4a and the twelfth accommodation side portion 4c12.

In addition, the twelfth accommodation side portion 4c12 can stably form a boundary where the visible shoes care device 1100 (or the article) is pushed to one side in the second direction (Y direction).

The thirteenth accommodation side portion 4c13 may be formed on the other side in the second direction (Y direction) with respect to the accommodation bottom portion 4a. The thirteenth accommodation side portion 4c13 may be formed in the first direction (X direction). As the thirteenth accommodation side portion 4c13 goes to the edge of the accommodation portion 4, that is, moves away from the accommodation bottom portion 4a to the other side in the second direction (Y direction), it may rise upwards in the third direction (Z direction).

In the process in which the user settles the visible shoes care device 1100 (or the article) on the accommodation bottom portion 4a, the thirteenth accommodation side portion 4c13 may form an inclined surface guiding the bottom surface of the visible shoes care device 1100 (or the article) to the accommodation bottom portion 4a, that is, to one side in the second direction (Y direction).

In addition, when the visible shoes care device 1100 (or the article) settled in the accommodation bottom portion 4a is pushed to the thirteenth accommodation side portion 4c13 due to the external force or the tilting of the shoes case device 1, the thirteenth accommodation side portion 4c13 may form a boundary where the visible shoes care device 1100 (or the article) is pushed to the other side in the second direction (Y direction) dur to the height difference from the accommodation bottom portion 4a.

Accordingly, various visible shoes care devices 1100 (or articles) settled in the accommodation bottom portion 4a can stably maintain the state of being settled in the accommodation bottom portion 4a.

In addition, the thirteenth accommodation side portion 4c13 may form a surface continuous with the surface of the accommodation bottom portion 4a. Further, as thirteenth accommodation side portion 4c13 moves to the edge of the accommodation portion, that is, gets away from the accommodation bottom portion 4a to the other side in the second direction (Y direction), the gradient may increase.

Accordingly, even if the visible shoes care device 1100 (or the article) settled in the accommodation bottom portion 4a is slightly pushed to the thirteenth accommodation side portion 4c13 due to the external force or the tilting of the shoes case device 1, the visible shoes care device 1100 (or article) may be prevented from being reversed due to the height difference between the accommodation bottom portion 4a and the thirteenth accommodation side portion 4c13.

In addition, the thirteenth accommodation side portion 4c13 can stably form a boundary where the visible shoes care device 1100 (or the article) is pushed to the other side in the second direction (Y direction).

The second accommodation side portion 4c2 may be formed on the upper surface of the door 30 along the edge of the accommodation portion 4. The second accommodation side portion 4c2 may form a surface connected to the accommodation extension portion 4b. The second accommodation side portion 4c2 may include a twenty-first accommodation side portion 4c21, a twenty-second accommodation side portion 4c22, and a twenty-third accommodation side portion 4c23.

The twenty-first accommodation side portion 4c21 may be formed in the front part in the first direction (X direction) with respect to the accommodation extension portion 4b. The twenty-first accommodation side portion 4c21 may be formed in the second direction (Y direction). As the twenty-first accommodation side portion 4c21 goes to the edge of the accommodation portion 4, that is, moves away from the accommodation extension portion 4b to the front part in the first direction (X direction), it may rise upwards in the third direction (Z direction).

As described above, the accommodation extension portion 4b may have the same height as the accommodation bottom portion 4a on a surface adjacent to the accommodation bottom portion 4a. Accordingly, with the door 30 closed, in the process in which the user settles the visible shoes care device 1100 (or the article) on the accommodation bottom portion 4a, the twenty-first accommodation side portion 4c21 may form an inclined surface guiding the bottom surface of the visible shoes care device 1100 (or the article) to the accommodation bottom portion 4a, that is, the rear part in the first direction (X direction), through the accommodation extension portion 4b.

In addition, with the door 30 closed, the visible shoes care device 1100 (or the article) on the accommodation bottom portion 4a is pushed to the twenty-first accommodation side portion 4c21 due to the external force or the tilting of the shoes care device 1, the twenty-first accommodation side portion 4c21 may form a boundary where the visible shoes care device 1100 (or the article) is pushed to the front part in the first direction (X direction) due to the height difference from the accommodation bottom portion 4a.

Accordingly, various the visible shoes care devices 1100 (or articles) settled in the accommodation bottom portion 4a may not drop outside the accommodation portion 4.

In addition, the twenty-first accommodation side portion 4c21 may form a surface continuous with the surface of the accommodation extension portion 4b. Further, the twenty-first accommodation side portion 4c21 moves to the edge of the accommodation portion 4, that is, gets way from the accommodation extension portion 4b to the front part in the first direction (X direction), the gradient may increase.

Accordingly, with the door 30 closed, even if the visible shoes care device 1100 (or the article) settled in the accommodation bottom portion 4a is slightly pushed to the twenty-first accommodation side portion 4c21 due to the external force or the tilting of the shoes care device 1, the visible shoes care device 1100 (or the article) can be prevented from being reversed due to the height difference between the accommodation extension portion 4b and the twenty-first accommodation side portion 4c21.

In addition, the twenty-first accommodation side portion 4c21 can stably form a boundary where the visible shoes care device 1100 (or the article) is pushed to the front side in the first direction (X direction).

The twenty-second accommodation side portion 4c22 may be formed on one side in the second direction (Y direction) with respect to the accommodation extension portion 4b. The twenty-second accommodation side portion 4c22 may be formed in the first direction (X direction). The twenty-second accommodation side portion 4c22 goes to the edge of the accommodation portion 4, that is, moves away from the accommodation extension portion 4b to one side in the second direction (Y direction), it may rise upwards in the third direction (Z direction). The twenty-second accommodation side portion 4c22 may form a surface continuous with the surface of the accommodation extension portion 4b.

The twenty-thirty accommodation side portion 4c23 may be disposed on another side in the second direction Y with reference to the accommodation extension portion. The second accommodation portion 4c23 may be provided along the first direction X. The twenty-thirty accommodation side portion 4c23 may be raised upward in the third direction Z in the direction toward the edge of the accommodation portion, i.e., in the direction away from the accommodation extension portion 4b to the other side in the second direction Y. The twenty-second accommodation portion 4c22 may provide a surface continuous with the surface of the accommodation extension portion 4b.

The door 30 may be hinge-coupled to the cabinet 3 around the rotation axis 31 in the vertical direction. As described above, the second concave groove 30h2 may be formed in the upper plate 30e. The user may pull and open the door 30 by applying force to the inner surface of the second concave groove 30h2. The second accommodation side portion 4c2 and the accommodation extension portion 4b may form the second concave groove 30h2.

The twenty-first accommodation side portion 4c21 may be formed in the front part in the first direction (X direction) with respect to the accommodation extension portion 4b. Accordingly, an action force of opening the door 30 may be applied to the door 30 through the second accommodation side portion 4c2.

As illustrated in FIG. 18A, the accommodation extension portion 4b may become lower toward the first direction (X direction). As described above, the twenty-first accommodation side portion 4c21 may form a surface continuous with the surface of the accommodation extension portion 4b. Accordingly, an area of the second accommodation side portion 4c2 to which the action force of opening the door 30 is applied may increase downwards in the third direction (Z direction).

Accordingly, the user's finger may form a wide contact surface with the second accommodation side portion 4c2. Accordingly, the action force of opening the door 30 may be dispersed on the contact surface. Therefore, when the door 30 is opened, as the pressure acting on a unit area of a finger surface decreases, the user can feel that the door 30 is easily opened.

The action force applied to the twenty-first accommodation side portion 4c21 through the user's finger may be divided into an X-axis component and a Z-axis component. A force applied to the eleventh accommodation side portion 4c11 through the bottom surface of the visible shoes care device 1100 (or the article) may also be divided into an X-axis component and a Z-axis component.

As illustrated in FIGS. 75a and 75b, the gradient of the second accommodation side portion 4c2 may be greater than the gradient of the first accommodation side portion 4c1 with respect to a direction parallel to the first direction (X direction). That is, the gradient of the twenty-first accommodation side portion 4c21 may be greater than the gradient of the eleventh accommodation side portion 4c11.

Accordingly, the action force applied to the twenty-first accommodation side portion 4c21 through the user's finger may be greater in the X-axis direction component than in the Z-axis direction component. Accordingly, the user may feel that the door 30 is easily opened.

In addition, the force applied to the eleventh accommodation side portion 4c11 through the bottom surface of the visible shoes case device 1100 (or the article) may be greater in the Z-axis direction component than in the X-axis direction component. Accordingly, while the bottom surface of the visible shoes case device 1100 (or the article) is in contact with the eleventh accommodation side portion 4c11, the possibility of reversing the visible shoes case device 1100 (or the article) can be reduced.

The first accommodation side portion 4c1 and the second accommodation side portion 4c2 may have the same height along the edge of the accommodation portion 4. Therefore, with the door 30 closed, the accommodation part 4 can be observed as an integral concave part having the accommodation side portion 4c of a certain height along the edge thereof, and the aesthetics of the upper surface of the shoes care device 1 can be improved.

An insertion portion 1140 that is convex downwards may be formed on the bottom surface of the main body 1110 such that it can be inserted into the accommodation portion 4. The insertion portion 1140 may be inserted into the accommodation portion 4 below the Z-axis. By inserting the insertion portion 1140 into the accommodation portion 4, the visible shoes care device 1100 may be stacked on the upper surface of the shoes care device 1.

The insertion portion 1140 may include a settling portion 1141, an insertion bottom portion 1142, and an insertion side portion 1143.

The settling portion 1141 is a component that can be settled in the accommodation bottom portion 4a, and is provided in a plural form. Three or more settling portions 1141 may be provided to prevent the reversing of the visible shoes care device 1100.

In one embodiment, four settling portions 1141 may be provided. The settling portions 1141 may protrude from the insertion bottom portion 1142 to a lower part of the Z-axis. The settling portions 1141 may form various shapes. In one embodiment, the settling portions 1141 may form a circular shape in a plan view.

The insertion bottom portion 1142 may be spaced apart upwards from the accommodation bottom portion 4a by the settling portion 1141. The insertion bottom portion 1142 may form a plane parallel to the horizontal plane. Accordingly, a gap between the insertion bottom portion 1142 and the accommodation bottom portion 4a in the Z-axis direction may be the same as the gap along the X-axis direction and the Y-axis direction. In one embodiment, the insertion bottom portion 1142 may have a substantially rectangular shape.

A dotted line of FIG. 19 may mean an edge of the insertion bottom portion 1142. The length of the insertion bottom portion 1142 with respect to the Y-axis direction may be substantially the same as the length of the accommodation bottom portion 4a.

The length of the insertion bottom portion 1142 may be smaller than the length of the accommodation bottom portion 4a with respect to the first direction (X direction). Accordingly, the settling portions 1141 protruding from the insertion bottom portion 1142 to the lower part of the Z-axis may be settled in the accommodation bottom portion 4a.

The insertion side portion 1143 may be formed along the edge of the insertion bottom portion 1142. The insertion side portion 1143 may be connected to the insertion bottom portion 1142. The insertion side portion 1143 may include a first insertion side portion 11431, a second insertion side portion 11432, a third insertion side portion 11433, and a fourth insertion side portion 11434.

The first insertion side portion 11431 may be formed in the rear part in the first direction (X direction) with respect to the insertion bottom portion 1142. The first insertion side portion 11431 may be formed in the second direction (Y direction). As the first insertion side portion 11431 goes to the edge of the insertion portion 1140, that is, moves away from the insertion bottom portion 1142 to the rear part in the first direction (X direction), it may rise upwards in the third direction (Z direction).

At least a part of the first insertion side portion 11431 may be lower than an upper end of the eleventh accommodation side portion 4c11. Accordingly, in the process in which the user settles the visible shoes care device 1100 (or the article) on the accommodation bottom portion 4a, the first insertion side portion 11431 may form an inclined surface corresponding to the eleventh accommodation side portion 4c11.

When the visible shoes care device 1100 (or the article) settled in the accommodation bottom portion 4a is pushed to the eleventh accommodation side portion 4c11 by the external force or the tilting of the shoes care device 1, the first insertion side portion 11431 may form an inclined surface in close contact with to the eleventh accommodation side portion 4c11.

When the visible shoes care device 1100 (or the article) settled in the accommodation bottom portion 4a is pushed to the eleventh accommodation side portion 4c11 by the external force or the tilting of the shoes care device 1, the first insertion side portion 11431 and the eleventh accommodation side portion 4c11 may form friction force. Accordingly, various visible shoes care devices 1100 (or articles) settled in the accommodation bottom portion 4a can stably maintain the state of being settled in the accommodation bottom portion 4a.

The second insertion side portion 11432 may be formed in the front part in the first direction (X direction) with respect to the insertion bottom portion 1142. The second insertion side portion 11432 may be formed in the second direction (Y direction). As the second insertion side portion 11432 goes to the edge of the insertion portion 1140, that is, moves away from the insertion bottom portion 1142 to the front part in the first direction (X direction), it may rise upwards in the third direction (Z direction).

The first insertion side portion 11431 and the second insertion side portion 11432 may form a symmetrical shape with respect to a reference plane RP' parallel to the second direction (Y direction) and the vertical direction.

That is, as shown in FIG. 13, when the visible shoes care device 1100 rotates 180 degrees around the Z-axis, the second insertion side portion 11432 may be located in the rear part in the first direction (X direction) with respect to the insertion bottom portion 1142.

Furthermore, the first insertion side portion 11431 may be positioned in the front part in the first direction (X direction) with respect to the insertion bottom portion 1142. In the process in which the user settles the visible shoes care device 1100 (or the article) in the accommodation bottom portion 4a, the second insertion side portion 11432 may form an inclined surface corresponding to the eleventh accommodation side portion 4c11.

When the visible shoes care device 1100 (or the article) settled in the accommodation bottom portion 4a is pushed to the eleventh accommodation side portion 4c11 by the external force or the tilting of the shoes care device 1, the second insertion side portion 11432 and the eleventh accommodation side portion 4c11 may form frictional force. Accordingly, various visible shoes care devices 1100 (or articles) settled in the accommodation bottom portion 4a can stably maintain the state of being settled in the accommodation bottom portion 4a.

The third insertion side portion 11433 may be formed on one side in the second direction (Y direction) with respect to the insertion bottom portion 1142. The third insertion side portion 11433 may be formed in the first direction (X direction). The third insertion side portion 11433 may form a vertical surface extending upwards in the Z-axis direction from the insertion bottom portion 1142. At least a part of the third insertion side portion 11433 may be higher than an upper end of the thirteenth accommodation side portion 4c13.

When the visible shoes care device 1100 (or the article) settled in the accommodation bottom portion 4a is pushed to the thirteenth accommodation side portion 4c13 by the external force or the tilting of the shoes care device 1, the third insertion side portion 11433 may form a surface in close contact with the thirteenth accommodation side portion 4c13. Accordingly, various visible shoes care devices 1100 (or articles) settled in the accommodation bottom portion 4a can stably maintain the state of being settled in the accommodation bottom portion 4a.

The fourth insertion side portion 11434 may be formed on the other side in the second direction (Y direction) with respect to the insertion bottom portion 1142. The fourth insertion side portion 11434 may be formed in the first direction (X direction). The fourth insertion side portion 11434 may form a vertical surface extending upwards in the Z-axis direction from the insertion bottom portion 1142. At least a part of the fourth insertion side portion 11434 may be higher than an upper end of the twelfth accommodation side portion 4c12.

When the visible shoes care device 1100 (or the article) settled in the accommodation bottom portion 4a is pushed to the twelfth accommodation side portion 4c12 by the external force or the tilting of the shoes care device 1, the fourth insertion side portion 11434 may form a surface in close contact with the twelfth accommodation side portion 4c12. Accordingly, various visible shoes care devices 1100 (or articles) settled in the accommodation bottom portion 4a can stably maintain the state of being settled in the accommodation bottom portion 4a.

As shown in FIGS. 74 and 75A, at least a part of the second accommodation side portion 4c2 may be disposed ahead of the visible shoes care device 1100 with respect to the first direction (X direction) in a state where the display space 1111 is closed.

As described above, the moving body 1120 may form a shape surrounding a lower part of the main body 1110. Accordingly, the moving body 1120 may be provided ahead of the main body 1110 in the first direction (X direction) under the main body 1110.

That is, with the display space 1111 closed, based on the first direction (X direction), at least a part of the second accommodation side portion 4c2 may be disposed ahead of the moving body 1120 in the first direction (X direction). That is, the second accommodation side portion 4c2 and the moving body 1120 may be spaced apart from each other in the first direction (X direction). The user may open the door 30 by inserting his/her finger into the second concave groove 30h2 through a gap between the second accommodation side portion 4c2 and the moving body 1120.

A separation extension portion 1150 may be formed on the bottom surface of the main body 1110. The separation extension portion 1150 may extend downwards in the Z-axis direction from the bottom surface of the main body 1110. The separation extension portion 1150 may separate the insertion portion 1140 from the bottom surface of the main body 1110 downwards in the Z-axis direction.

The insertion portion 1140 may be spaced apart from the bottom surface of the main body 1110 downwards in the Z-axis direction by the separation extension portion 1150. The separation extension portion 1150 and the insertion side portion 1143 may be connected to each other.

The moving body 1120 and the main body 1110 may be spaced apart from the second accommodation side portion 4c2 in the Z-axis direction above the door 30. That is, a gap (or an interval) between the second accommodation side portion 4c2 and the moving body 1120 may be formed in the X-axis direction and the Z-axis direction.

Accordingly, the gap (or the interval) between the second accommodation side portion 4c2 and the moving body 1120 may extend in a direction approximately perpendicular to the direction in which the user's finger enters. The user can open the door 30 by easily inserting his/her finger into the second concave groove 30h2 through the gap between the second accommodation side portion 4c2 and the moving body 1120.

In addition, as the moving body 1120 and the main body 1110 are spaced apart from the second accommodation side portion 4c2 in the Z-axis direction above the door 30, the moving body 1120 can open and close the display space 1111 in a direction parallel to the first direction (X direction) above the insertion portion 1140 and the accommodation portion 4. Accordingly, even if the visible shoes care device 1100 is stacked on the upper surface of the shoes care device 1, the visible shoes care device 1100 may not interfere with the opening and closing of the door 30.

Accordingly, the user may separately open or simultaneously open the door 30 of the shoes care device 1 and the movable body 1120 of the visible shoes care device 1100. Therefore, even if the visible shoes care device 1100 is stacked on the upper surface of the shoes care device 1, the shoes care device 1 and the visible shoes care device 1100 may be conveniently used without interfering with each other.

Hereinabove, a specific embodiment of the present invention is described and illustrated, but the present invention is not limited to the disclosed embodiment, and it may be appreciated by those skilled in the art that the exemplary embodiment can be variously modified and transformed to another specific embodiment without departing from the spirit and the scope of the present invention. Therefore, the scope of the present invention will not be defined by the described embodiment, but defined by the technical spirit disclosed in the claims.

### [Industrial Applicability]

With the shoe care device according to the present disclosure, in view of the points that it is possible to stack articles on the upper surface of the shoe care device, and that the upper surfaces of the cabinet and the door that constitute the upper surface of the shoe care device are configured to provide interior aesthetics, the present disclosure overcomes the limitations of existing technology and can be used in related technologies, the possibility of marketing or commercializing devices to which the present disclosure is applied is sufficient, and it is apparent that the present disclosure can be implemented in reality. Therefore, the present disclosure has industrial applicability.

## Claims

1. A shoe care device (1) configured to provide therein an accommodation space (101) through which air is forced to circulate to treat the shoes (S), the shoe care device (1) comprising:
a downwardly concave accommodation portion (4) provided on an upper surface of the shoe care device (1);
a cabinet (20) having a main opening (140) that opens the accommodation space (101) in a horizontal first direction (X); and
a door (30) configured to open and close the main opening (140) and defining the accommodation portion (4) together with the cabinet (20),
wherein the accommodation portion (4) comprises:
an accommodation bottom portion (4a) provided on an upper surface of the cabinet (20), wherein the accommodation bottom portion (4a) is configured such that an article can be seated thereon, **characterized in that** the shoe care device (1) further comprises
an accommodation extension portion (4b) provided on an upper surface of the door (30) wherein a surface thereof adjacent to the accommodation bottom portion (4a) has a height equal to that of the accommodation bottom portion (4a); and
an accommodation side portion (4c) defining an edge of the accommodation portion (4) and being higher than the accommodation bottom portion (4a).

2. The shoe care device (1) of claim 1, wherein the accommodation side portion (4c) comprises:
a first accommodation side portion (4c1) provided on the upper surface of the cabinet (20) and connected to the accommodation bottom portion (4a); and
a second accommodation side portion (4c2) provided on the upper surface of the door (30) and connected to the accommodation extension portion (4b),
wherein the door (30) is hinged to the cabinet (20) about a vertical rotation axis (31), and
wherein an action force of opening the door (30) is applied to the door (30) through the second accommodation side portion (4c2).

3. The shoe care device (1) of claim 2, wherein the accommodation extension portion (4b) has a height that is lowered in the first direction (X) such that an area of the second accommodation side portion (4c2) to which the action force is applied increases.

4. The shoe care device (1) of claim 2 or 3, wherein, with reference to a direction parallel to the first direction (X), a gradient of the second accommodation side portion (4c2) is greater than that of the first accommodation side portion (4c1).

5. The shoe care device (1) of claim 2, wherein the first accommodation side portion (4c1) and the second accommodation side portion (4c2) have an equal height along the edge of the accommodation portion (4).

6. The shoe care device (1) of one of claims 1 to 5, wherein the accommodation portion (4) has a symmetrical shape with respect to a reference plane (RP) parallel to the first direction (X) and the vertical direction.

7. A shoe care set (1000) comprises:
a shoe care device (1) configured to provide therein an accommodation space (101) in which air is forced to circulate to treat shoes (S); and
a visible shoe care device (1100) configured to be stacked on an upper surface of the shoe care device (1), wherein the visible shoe care device (1100) is configured to define a display space (1111) therein to display the shoes (S),
**characterized in that**
a downwardly concave accommodation portion (4) is provided on an upper surface of the shoe care device (1), and
wherein an insertion portion (1140) that is convex downward may be provided on a bottom surface of the visible shoe care device (1100) such that the insertion portion (1140) can be inserted into the accommodation portion (4).

8. The shoe care set (1000) of claim 7, wherein the shoe care device (1) comprises:
a cabinet (20) having a main opening (140) that opens the accommodation space (101) in a horizontal first direction (X); and
a door (30) configured to open and close the main opening (140) and defining the accommodation portion (4) together with the cabinet (20),
wherein the accommodation portion (4) comprises:
an accommodation bottom portion (4a) provided on an upper surface of the cabinet (20), wherein the accommodation bottom portion (4a) is configured such that the insertion portion (1140) can be seated thereon; and
an accommodation extension portion (4b) provided on an upper surface of the door (30) wherein a surface thereof adjacent to the accommodation bottom portion (4a) has a height equal to that of the accommodation bottom portion (4a).

9. The shoe care set (1000) of claim 8, further comprising:
an accommodation side portion (4c) that is higher than the accommodation bottom portion (4a) to prevent separation of the insertion portion (1140) from the accommodation bottom portion (4a),
wherein the accommodation side portion (4c) comprises:
a first accommodation side portion (4c1) provided on the upper surface of the cabinet (20) and connected to the accommodation bottom portion (4a); and
a second accommodation side portion (4c2) provided on the upper surface of the door (30) and connected to the accommodation extension portion (4b), and
wherein an action force of opening the door (30) is applied to the door (30) through the second accommodation side portion (4c2).

10. The shoe care set (1000) of claim 9, wherein the accommodation extension portion (4b) has a height that is lowered in the first direction (X) such that an area of the second accommodation side portion (4c2) to which the action force is applied increases.

11. The shoe care set (1000) of claim 9 or 10, wherein, in a state in which the visible shoe care device (1100) closed, at least a portion of the second accommodation side portion (4c2) is located in front of the visible shoe care device (1100) with reference to the first direction (X).

12. The shoe care set (1000) of one of claims 9 to 11, wherein the visible shoe care device (1100) comprises:
a main body (1110) providing the insertion portion (1140) and the display space (1111); and
a moving body (1120) coupled to the main body (1110) to be slidable in the first direction (X) to open the visible shoe care device (1100), and
wherein the moving body (1120) is configured to open and close the visible shoe care device (1100) in a direction parallel to the first direction (X) above the insertion portion (1140) and the accommodation portion (4).

13. The shoe care set (1000) of claim 12, wherein the moving body (1120) and the main body (1110) are spaced apart from the second accommodation side portion (4c2) above the door (30).

14. The shoe care set (1000) of claim 12 or 13, wherein the moving body (1120) comprises a transparent window (1130).

15. The shoe care set (1000) of one of claims 9 to 14, wherein the insertion portion (1140) comprises:
a plurality of seating portions (1141) configured to be seated on the accommodation bottom portion (4a);
an insertion bottom portion (1142) spaced upward from the accommodation bottom portion (4a) by the seating portions (1141); and
an insertion side portion (1143) provided along an edge of the insertion bottom portion (1142), wherein at least a portion of the insertion side portion (1143) has a height lower than an upper end of the accommodation side portion (4c).

## Patentansprüche

1. Schuhpflegevorrichtung (1), die dazu eingerichtet ist, einen Aufnahmeraum (101) bereitzustellen, durch den Luft zum Zirkulieren gebracht wird, um die Schuhe (S) zu behandeln, wobei die Schuhpflegevorrichtung (1) umfasst:
einen nach unten konkaven Aufnahmeabschnitt (4), der an einer Oberseite der Schuhpflegevorrichtung (1) vorgesehen ist;
einen Schrank (20) mit einer Hauptöffnung (140), die den Aufnahmeraum (101) in einer horizontalen ersten Richtung (X) öffnet; und
eine Tür (30), die dazu eingerichtet ist, die Hauptöffnung (140) zu öffnen und zu schließen und zusammen mit dem Gehäuse (20) den Aufnahmeabschnitt (4) zu definieren,
wobei der Aufnahmeabschnitt (4) umfasst:
einen Aufnahmebodenabschnitt (4a), der an einer Oberseite des Schranks (20) vorgesehen ist, wobei der Aufnahmebodenabschnitt (4a) dazu eingerichtet ist, dass ein Gegenstand darauf abgestellt werden kann,
**dadurch gekennzeichnet, dass** die Schuhpflegevorrichtung (1) ferner umfasst
einen Aufnahmeverlängerungsabschnitt (4b), der an einer Oberseite der Tür (30) vorgesehen ist, wobei eine Oberfläche davon, die an den Aufnahmebodenabschnitt (4a) angrenzt, eine Höhe aufweist, die der des Aufnahmebodenabschnitts (4a) entspricht; und
einen Aufnahmeseitenabschnitt (4c), der eine Kante des Aufnahmeabschnitts (4) definiert und höher als der Aufnahmebodenabschnitt (4a) ist.

2. Schuhpflegevorrichtung (1) nach Anspruch 1, wobei der Aufnahmeseitenabschnitt (4c) umfasst:
einen ersten Aufnahmeseitenabschnitt (4c1), der an der Oberseite des Schranks (20) vorgesehen und mit dem Aufnahmebodenabschnitt (4a) verbunden ist; und
einen zweiten Aufnahmeseitenabschnitt (4c2), der an der Oberseite der Tür (30) vorgesehen und mit dem Aufnahmeverlängerungsabschnitt (4b) verbunden ist,
wobei die Tür (30) um eine vertikale Drehachse (31) an dem Schrank (20) angelenkt ist, und
wobei eine Betätigungskraft zum Öffnen der Tür (30) über den zweiten Aufnahmeseitenabschnitt (4c2) auf die Tür (30) ausgeübt wird.

3. Schuhpflegevorrichtung (1) nach Anspruch 2, wobei der Aufnahmeverlängerungsabschnitt (4b) eine Höhe aufweist, die in der ersten Richtung (X) so abgesenkt ist, dass eine Wirkfläche des zweiten Aufnahmeseitenabschnitts (4c2), auf die die Betätigungskraft ausgeübt wird, vergrößert wird.

4. Schuhpflegevorrichtung (1) nach Anspruch 2 oder 3, wobei in Bezug auf eine Richtung parallel zur ersten Richtung (X) die Neigung des zweiten Aufnahmeseitenabschnitts (4c2) größer ist als die des ersten Aufnahmeseitenabschnitts (4c1).

5. Schuhpflegevorrichtung (1) nach Anspruch 2, wobei der erste Aufnahmeseitenabschnitt (4c1) und der zweite Aufnahmeseitenabschnitt (4c2) entlang der Kante des Aufnahmeabschnitts (4) eine gleiche Höhe aufweisen.

6. Schuhpflegevorrichtung (1) nach einem der Ansprüche 1 bis 5, wobei der Aufnahmeabschnitt (4) eine symmetrische Form in Bezug auf eine Bezugsebene (RP) parallel zur ersten Richtung (X) und zur vertikalen Richtung aufweist.

7. Schuhpflegeset (1000) umfassend:
eine Schuhpflegevorrichtung (1), die dazu eingerichtet ist, einen Aufnahmeraum (101) bereitzustellen, durch den Luft zum Zirkulieren gebracht wird, um die Schuhe (S) zu behandeln; und
eine sichtbare Schuhpflegevorrichtung (1100), die dazu eingerichtet ist, auf einer Oberseite der Schuhpflegervorrichtung (1) gestapelt werden zu können, wobei die sichtbare Schuhpflegervorrichtung (1100) dazu eingerichtet ist, darin einen Präsentationsraum (1111) zu definieren, um die Schuhe (S) zu präsentieren,
**dadurch gekennzeichnet, dass**
ein nach unten konkaver Aufnahmeabschnitt (4) auf einer Oberseite der Schuhpflegevorrichtung (1) vorgesehen ist, und
wobei ein nach unten konvexer Einlegeabschnitt (1140) an einer Unterseite der sichtbaren Schuhpflegevorrichtung (1100) vorgesehen sein kann, so dass der Einlegeabschnitt (1140) in den Aufnahmeabschnitt (4) eingeführt werden kann.

8. Schuhpflegeset (1000) nach Anspruch 7, wobei die Schuhpflegevorrichtung (1) umfasst:
einen Schrank (20) mit einer Hauptöffnung (140), die den Aufnahmeraum (101) in einer horizontalen ersten Richtung (X) öffnet; und
eine Tür (30), die dazu eingerichtet ist, die Hauptöffnung (140) zu öffnen und zu schließen und zusammen mit dem Schrank (20) den Aufnahmeabschnitt (4) zu definieren,
wobei der Aufnahmeabschnitt (4) umfasst:
einen Aufnahmebodenabschnitt (4a), der an einer Oberseite des Schranks (20) vorgesehen ist, wobei der Aufnahmebodenabschnitt (4a) dazu eingerichtet ist, dass der Einlegeabschnitt (1140) darauf aufgelegt werden kann; und
einen Aufnahmeverlängerungsabschnitt (4b), der auf einer Oberseite der Tür (30) vorgesehen ist, wobei eine Oberfläche davon, die an den Aufnahmebodenabschnitt (4a) angrenzt, eine Höhe aufweist, die der des Aufnahmebodenabschnitts (4a) entspricht.

9. Schuhpflegeset (1000) nach Anspruch 8, das ferner umfasst:
einen Aufnahmeseitenabschnitt (4c), der höher als der Aufnahmebodenabschnitt (4a) ist, um ein Trennen des Einlegeabschnitts (1140) von dem Aufnahmebodenabschnitt (4a) zu verhindern,
wobei der Aufnahmeseitenabschnitt (4c) umfasst:
einen ersten Aufnahmeseitenabschnitt (4c1), der an der Oberseite des Schranks (20) vorgesehen und mit dem Aufnahmebodenabschnitt (4a) verbunden ist; und
einen zweiten Aufnahmeseitenabschnitt (4c2), der an der Oberseite der Tür (30) vorgesehen und mit dem Aufnahmeverlängerungsabschnitt (4b) verbunden ist, und
wobei eine Betätigungskraft zum Öffnen der Tür (30) über den zweiten Aufnahmeseitenabschnitt (4c2) auf die Tür (30) ausgeübt wird.

10. Schuhpflegeset (1000) nach Anspruch 9, wobei der Aufnahmeverlängerungsabschnitt (4b) eine Höhe aufweist, die in der ersten Richtung (X) so abgesenkt ist, dass eine Wirkfläche des zweiten Aufnahmeseitenabschnitts (4c2), auf die die Betätigungskraft ausgeübt wird, vergrößert wird

11. Schuhpflegeset (1000) nach Anspruch 9 oder 10, wobei in einem Zustand, in dem die sichtbare Schuhpflegevorrichtung (1100) geschlossen ist, mindestens ein Teil des zweiten Aufnahmeseitenabschnitts (4c2) in Bezug auf die erste Richtung (X) vor der sichtbaren Schuhpflegevorrichtung (1100) angeordnet ist.

12. Schuhpflegeset (1000) nach einem der Ansprüche 9 bis 11, wobei die sichtbare Schuhpflegevorrichtung (1100) umfasst:
einen Hauptkörper (1110), der den Einlegeabschnitt (1140) und den Präsentationsraum (1111) bereitstellt; und
einen Bewegungskörper (1120), der mit dem Hauptkörper (1110) verbunden ist, um in der ersten Richtung (X) verschiebbar zu sein, um die sichtbare Schuhpflegevorrichtung (1100) zu öffnen, und
wobei der Bewegungskörper (1120) dazu eingerichtet ist, die sichtbare Schuhpflegevorrichtung (1100) in einer Richtung parallel zur ersten Richtung (X) über dem Einlegeabschnitt (1140) und dem Aufnahmeabschnitt (4) zu öffnen und zu schließen.

13. Schuhpflegeset (1000) nach Anspruch 12, wobei der Bewegungskörper (1120) und der Hauptkörper (1110) von dem zweiten Aufnahmeseitenabschnitt (4c2) oberhalb der Tür (30) beabstandet sind.

14. Schuhpflegeset (1000) nach Anspruch 12 oder 13, wobei der Bewegungskörper (1120) ein transparentes Fenster (1130) umfasst.

15. Schuhpflegeset (1000) nach einem der Ansprüche 9 bis 14, wobei der Einlegeabschnitt (1140) umfasst:
eine Mehrzahl von Sitzabschnitten (1141), die dazu eingerichtet sind, auf dem Aufnahmebodenabschnitt (4a) zu sitzen;
einen Einführungsbodenabschnitt (1142), der durch die Sitzabschnitte (1141) nach oben von dem Aufnahmebodenabschnitt (4a) beabstandet ist; und
einen Einführungsseitenabschnitt (1143), der entlang einer Kante des Einführungsbodenabschnitts (1142) vorgesehen ist, wobei mindestens ein Teil des Einführungsseitenabschnitts (1143) eine Höhe aufweist, die geringer ist als ein oberes Ende des Aufnahmeseitenabschnitts (4c).

## Revendications

1. Dispositif d'entretien de chaussures (1) conçu pour fournir un espace de logement (101) à travers lequel l'air est forcé à circuler pour traiter les chaussures (S), le dispositif d'entretien de chaussures (1) comprenant:
une partie de logement concave vers le bas (4) disposée sur une surface supérieure du dispositif d'entretien de chaussure (1);
une armoire (20) dotée d'une ouverture principale (140) qui ouvre l'espace de logement (101) dans une première direction horizontale (X); et
une porte (30) conçue pour ouvrir et fermer l'ouverture principale (140) et formant conjointement avec l'armoire (20) la partie de logement (4),
dans lequel la partie de logement (4) comprend:
une partie inférieure de logement (4a) disposée sur une surface supérieure de l'armoire (20), la partie inférieure de logement (4a) est conçue de manière à pouvoir recevoir un article, **caractérisé en ce que** le dispositif d'entretien de chaussures (1) comprend en outre
une partie étendue de logement (4b) disposée sur une surface supérieure de la porte (30), dans lequel sa surface adjacente à la partie inférieure de logement (4a) a une hauteur égale à celle de la partie inférieure de logement (4a); et
une partie latérale de logement (4c) formant un bord de la partie de logement (4) et étant plus haute que la partie inférieure de logement (4a).

2. Dispositif d'entretien de chaussures (1) selon la revendication 1, dans lequel la partie latérale de logement (4c) comprend:
une première partie latérale de logement (4c1) disposée sur la surface supérieure de l'armoire (20) et reliée à la partie inférieure de logement (4a); et
une seconde partie latérale de logement (4c2) disposée sur la surface supérieure de la porte (30) et reliée à la partie étendue de logement (4b),
dans lequel la porte (30) est articulée sur l'armoire (20) autour d'un axe de rotation vertical (31), et
dans lequel une force d'ouverture (30) est appliquée à la porte (30) par l'intermédiaire de la seconde partie latérale de logement (4c2).

3. Dispositif d'entretien de chaussures (1) selon la revendication 2, dans lequel la hauteur de la partie étendue de logement (4b) est abaissée dans la première direction (X) de telle sorte qu'une zone de la seconde partie latérale de logement (4c2) à laquelle la force d'action est appliquée augmente.

4. Dispositif d'entretien de chaussures (1) selon la revendication 2 ou 3, dans lequel par rapport à une direction parallèle à la première direction (X), le gradient de la seconde partie latérale de logement (4c2) est supérieur à celui de la première partie latérale de logement (4c1).

5. Dispositif d'entretien de chaussures (1) selon la revendication 2, dans lequel la première partie latérale de logement (4c1) et la seconde partie latérale de logement (4c2) ont une hauteur égale le long du bord de la partie de logement (4).

6. Dispositif d'entretien de chaussures (1) selon l'une quelconque des revendications 1 à 5, dans lequel la partie de logement (4) présente une forme symétrique par rapport à un plan de référence (RP) parallèle à la première direction (X) et à la direction verticale.

7. Ensemble d'entretien de chaussures (1000) comprenant:
dispositif d'entretien de chaussures (1) conçu pour fournir un espace de logement (101) à travers lequel l'air est forcé à circuler pour traiter les chaussures (S); et
un dispositif visible d'entretien des chaussures (1100) configuré pour être placé sur le dessus du dispositif d'entretien de chaussures (1), dans lequel le dispositif visible d'entretien de chaussures (1100) est conçu pour former un espace de présentation (1111) pour présenter les chaussures (S),
**caractérisé en ce qu'**une partie de logement concave vers le bas (4) est disposée sur une surface supérieure du dispositif d'entretien de chaussure (1); et
dans lequel une partie d'insertion (1140) qui est convexe vers le bas peut être disposée sur une surface inférieure du dispositif visible d'entretien de chaussures (1100) de telle sorte que la partie d'insertion (1140) puisse être insérée dans la partie de logement (4).

8. Ensemble d'entretien de chaussures (1000) selon la revendication 7, dans lequel le dispositif d'entretien de chaussures (1) comprend:
une armoire (20) dotée d'une ouverture principale (140) qui ouvre l'espace de logement (101) dans une première direction horizontale (X); et
une porte (30) conçue pour ouvrir et fermer l'ouverture principale (140) et formant conjointement avec l'armoire (20) la partie de logement (4),
dans lequel la partie de logement (4) comprend:
une partie inférieure de logement (4a) disposée sur une surface supérieure de l'armoire (20), la partie inférieure de logement (4a) est conçue de manière à ce que la partie d'insertion (1140) puisse être placée dessus; et
une partie étendue de logement (4b) disposée sur une surface supérieure de la porte (30), dans lequel sa surface adjacente à la partie inférieure de logement (4a) a une hauteur égale à celle de la partie inférieure de logement (4a).

9. Ensemble d'entretien de chaussures (1000) selon la revendication 8, comprenant en outre:
une partie latérale de logement (4c) qui est plus haute que la partie inférieure de logement (4a) afin d'empêcher la séparation de la partie d'insertion (1140) de la partie inférieure de logement (4a),
dans lequel la seconde partie latérale de logement (4c) comprend:
une première partie latérale de logement (4c1) disposée sur la surface supérieure de l'armoire (20) et reliée à la partie inférieure de logement (4a); et
une seconde partie latérale de logement (4c2) disposée sur la surface supérieure de la porte (30) et reliée à la partie étendue de logement (4b), et
dans lequel une force d'ouverture (30) est appliquée à la porte (30) par l'intermédiaire de la seconde partie latérale de logement (4c2).

10. Ensemble d'entretien de chaussures (1) selon la revendication 9, dans lequel la partie d'extension d'adaptation (4b) a une hauteur qui est abaissée dans la première direction (X) de telle sorte qu'une zone de la deuxième partie latérale d'adaptation (4c2) à laquelle la force d'action est appliquée augmente.

11. Ensemble d'entretien de chaussures (1000) selon la revendication 9 ou 10, lorsque le dispositif visible d'entretien de chaussures (1100) est fermé, au moins une partie de la seconde partie latérale de logement (4c2) est située devant le dispositif visible d'entretien de chaussures (1100) par rapport à la première direction (X).

12. Ensemble d'entretien de chaussures (1000) selon l'une quelconque des revendications 9 à 11, dans lequel le dispositif d'entretien de chaussures (1100) comprend:
un corps principal (1110) comportant la partie d'insertion (1140) et l'espace de présentation (1111); et
un corps mobile (1120) couplé au corps principal (1110) pour pouvoir coulisser dans la première direction (X) afin d'ouvrir le dispositif visible d'entretien de chaussures (1100), et
dans lequel le corps mobile (1120) est conçu pour ouvrir et fermer le dispositif visible d'entretien de chaussures (1100) dans une direction parallèle à la première direction (X) au-dessus de la partie d'insertion (1140) et de la partie de logement (4).

13. Ensemble d'entretien de chaussures (1000) selon la revendication 12, dans lequel le corps mobile (1120) et le corps principal (1110) sont espacés de la deuxième partie latérale de logement (4c2) au-dessus de la porte (30).

14. Ensemble d'entretien de chaussures (1000) selon la revendication 12 ou 13, dans lequel le corps mobile (1120) comprend une fenêtre transparente (1130).

15. Ensemble d'entretien de chaussures (1000) selon l'une quelconque des revendications 9 à 14, dans lequel la partie d'insertion (1140) comprend:
une pluralité de parties d'appui (1141) conçues pour être placées sur la partie inférieure d'accueil (4a);
une partie inférieure d'insertion (1142) espacée vers le haut de la partie inférieure de logement (4a) par les parties d'appui (1141); et
une partie latérale d'insertion (1143) disposée le long d'un bord de la partie inférieure d'insertion (1142), dans lequel au moins une partie de la partie latérale d'insertion (1143) a une hauteur inférieure à une extrémité supérieure de la partie latérale de logement (4c).
